# EUROPEAN PATENT APPLICATION

(11) **EP 4 471 072 A1**
(43) Date of publication of application: **04.12.2024**
(21) Application number: 23746732.9
(22) Date of filing: 16.01.2023
(51) Int. Cl.: C08G 18/32, A61K 8/87, A61Q 1/04, A61Q 1/10, A61Q 1/12, A61Q 5/02, A61Q 5/06, A61Q 5/12, A61Q 17/04, C08G 18/75

(54) **COPOLYMER AND COSMETIC COMPOSITION CONTAINING SAID COPOLYMER**

(30) Priority: 25.01.2022 JP 2022009220
(71) Applicant: ADEKA CORPORATION, Arakawa-ku Tokyo 116-8554 (JP)
(72) Inventor: YASUTANI, Akihito, Tokyo 116-8554 (JP); MAEBASHI, Marika, Tokyo 116-8554 (JP); TAKEISHI, Yuki, Tokyo 116-8554 (JP); KONDO, Yoshihide, Tokyo 116-8554 (JP)
(74) Representative: Müller-Boré & Partner Patentanwälte PartG mbB
(86) International application number: PCT/JP2023/001028
(87) International publication number: WO 2023/145518

(57) **Abstract**

Disclosed herein are: a copolymer obtained by causing a diglycerin fatty acid ester represented by the following general formula (1) and isophorone diisocyanate to react with each other, the copolymer having a weight-average molecular weight of from 3,500 to 100,000; and a cosmetic composition including the copolymer: 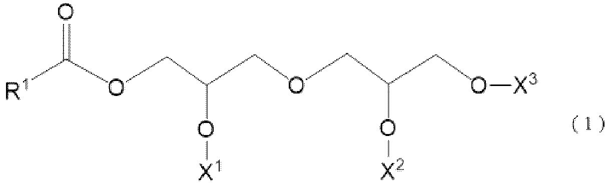 where R¹ represents an alkyl group having 8 to 24 carbon atoms or an alkenyl group having 8 to 24 carbon atoms, X¹ to X³ each independently represent a hydrogen atom or a group represented by -C(=O)R², and R² represents an alkyl group having 8 to 24 carbon atoms or an alkenyl group having 8 to 24 carbon atoms, provided that at least one of X¹ to X³ represents a hydrogen atom.

## Description

### Technical Field

The present invention relates to a copolymer that can improve various characteristics, such as applicability, an adhesive feeling, a glossy feeling, and usability, of various products, and that can be particularly suitably used as a raw material of a cosmetic preparation, and to a cosmetic composition containing the copolymer.

### Background Art

Polyurethanes having various structures have heretofore been developed through utilization of, for example, flexibility, elasticity, strength, stability, and safety of their particles or coating films, and the polyurethanes have been used for products, such as a cosmetic preparation and a coating agent. For example, in Patent Document 1, there is a description of an organic fine spherical powder-containing cosmetic preparation containing specific spherical polyurethane fine powder, the organic fine spherical powder-containing cosmetic preparation having excellent lubricity and touch feeling on skin. In Patent Document 2, there is a proposal of an aerosol composition consisting of an aqueous stock solution containing a urethane resin and a liquefied gas, the aerosol composition being useful as an aerosol-type gommage cosmetic preparation. In Patent Document 3, there is a proposal of a use of a salt of polyurethane consisting of a polylactic acid polyol, a diol, and a diisocyanate as an aid in preparation of a cosmetic and a drug.

In addition, in Patent Document 4, as an oily composition, which easily adjusts the viscosity of a product, and imparts a satisfactory touch feeling and a satisfactory thickening effect to the product, there is a description of an oily composition, comprising: at least one kind of urethane polymer selected from the group consisting of: a (PPG-12/SMDI) copolymer; and a (polyglyceryl-2 diisostearate/IPDI) copolymer; silica particles; and one or more kinds of oil components selected from an ester oil, a hydrocarbon oil, and a silicone oil. When the oily composition is used, a satisfactory touch feeling and a satisfactory thickening effect can be imparted to the product. However, the oily composition is difficult to be applied in some cases depending on a form of a final product because the oily composition comprises the silica particles as an essential constituent for imparting the characteristics. In addition, there has been a risk in that the oily composition adversely affects, for example, a glossy feeling or usability in a cosmetic preparation. Further, with regard to the touch feeling or the like to be obtained, the level required by users has not been satisfied yet. Accordingly, in the market, there has been required development of a raw material that has a simpler configuration and improves various characteristics, such as applicability, an adhesive feeling, a glossy feeling, and usability, of various products.

### Citation List

### Patent Document

[Patent Document 1] JP 5-262622 A
[Patent Document 2] JP 2003-137730 A
[Patent Document 3] JP 7-509741 A
[Patent Document 4] WO 2021/182500 A1

### Summary of Invention

### Technical Problem

Accordingly, an object of the present invention is to provide: a copolymer that can improve various characteristics, such as applicability, an adhesive feeling, a glossy feeling, and usability, of various products, and that can be particularly suitably used as a raw material of a cosmetic preparation; and a cosmetic composition containing the copolymer.

### Solution to Problem

In view of the foregoing, the inventors of the present invention have made extensive investigations, and as a result, have found that a specific copolymer is excellent in all of applicability, adhesive feeling, glossy feeling, and usability, and hence can be particularly suitably used for a cosmetic preparation. Thus, the inventors have reached the present invention. That is, according to an embodiment of the present invention, there is provided a copolymer obtained by causing a diglycerin fatty acid ester represented by the following general formula (1) and isophorone diisocyanate to react with each other, the copolymer having a weight-average molecular weight of from 3,500 to 100,000.

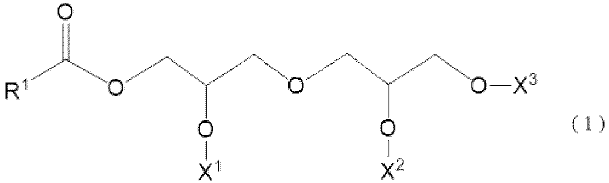

In the general formula (1), R¹ represents an alkyl group having 8 to 24 carbon atoms or an alkenyl group having 8 to 24 carbon atoms, X¹ to X³ each independently represent a hydrogen atom or a group represented by -C(=O)R², and R² represents an alkyl group having 8 to 24 carbon atoms or an alkenyl group having 8 to 24 carbon atoms, provided that at least one of X¹ to X³ represents a hydrogen atom.

### Advantageous Effects of Invention

The copolymer of the present invention is used as a raw material of various products, improves various characteristics, such as applicability, an adhesive feeling, a glossy feeling, and usability, of the products at the same time, and can be particularly suitably used as a raw material of a cosmetic preparation.

### Description of Embodiments

A diglycerin fatty acid ester to be used in the production of a copolymer of the present invention is a diglycerin fatty acid ester represented by the following general formula (1).

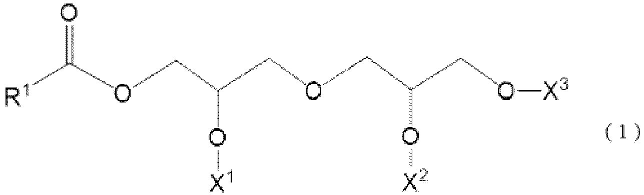

In the general formula (1), R¹ represents an alkyl group having 8 to 24 carbon atoms or an alkenyl group having 8 to 24 carbon atoms. Examples of such group include a linear alkyl group having 8 to 24 carbon atoms, a branched alkyl group having 8 to 24 carbon atoms, a linear alkenyl group having 8 to 24 carbon atoms, and a branched alkenyl group having 8 to 24 carbon atoms.

Examples of the above-mentioned alkyl group include an octyl group, a 2-ethylhexyl group, a secondary octyl group, a nonyl group, a secondary nonyl group, a decyl group, a secondary decyl group, an undecyl group, a secondary undecyl group, a dodecyl group, a secondary dodecyl group, a tetradecyl group, a secondary tetradecyl group, a hexadecyl group, a secondary hexadecyl group, a stearyl group, an eicosyl group, a docosyl group, a tetracosyl group, a 2-butyloctyl group, a 2-butyldecyl group, a 2-hexyloctyl group, a 2-hexyldecyl group, a 2-octyldecyl group, a 2-hexyldodecyl group, a 2-octyldodecyl group, a 2-decyltetradecyl group, and an isostearyl group.

An example of the alkenyl group is an alkenyl group obtained by substituting a methylene group at any position in the above-mentioned alkyl group with -CH=CH-.

Of those, R¹ represents preferably an alkyl group having 10 to 22 carbon atoms or an alkenyl group having 10 to 22 carbon atoms, more preferably an alkyl group having 12 to 20 carbon atoms or an alkenyl group having 12 to 20 carbon atoms, still more preferably a linear or branched alkyl group having 12 to 18 carbon atoms, particularly preferably a branched alkyl group having 12 to 18 carbon atoms from the viewpoints of the applicability and adhesive feeling of each of the copolymer and a cosmetic composition to be obtained. R¹ may represent, for example, an isostearyl group.

In the general formula (1), X¹ to X³ each independently represent a hydrogen atom or a group represented by -C(=O)R². However, at least one of X¹ to X³ represents a hydrogen atom. In particular, it is preferred that two of X¹ to X³ each represent a hydrogen atom and the other represent a group represented by -C(=O)R², and it is more preferred that X¹ and X² each represent a hydrogen atom and X³ represent a group represented by -C(=O)R² from the viewpoints of the applicability and adhesive feeling of each of the copolymer and cosmetic composition to be obtained.

R² represents an alkyl group having 8 to 24 carbon atoms or an alkenyl group having 8 to 24 carbon atoms. Examples of such group include a linear alkyl group having 8 to 24 carbon atoms, a branched alkyl group having 8 to 24 carbon atoms, a linear alkenyl group having 8 to 24 carbon atoms, and a branched alkenyl group having 8 to 24 carbon atoms. Specific examples of the alkyl group or the alkenyl group represented by R² include the same groups as those of the above-mentioned R¹. Of those, R² represents preferably an alkyl group having 10 to 22 carbon atoms or an alkenyl group having 10 to 22 carbon atoms, more preferably an alkyl group having 12 to 20 carbon atoms or an alkenyl group having 12 to 20 carbon atoms, still more preferably a linear or branched alkyl group having 12 to 18 carbon atoms, particularly preferably a branched alkyl group having 12 to 18 carbon atoms from the viewpoints of the applicability and adhesive feeling of each of the copolymer and cosmetic composition to be obtained. In the present invention, when two or more of X¹ to X³ each represent a group represented by -C(=O)R², the respective R²s may represent identical groups or different groups. R² may represent, for example, an isostearyl group. In addition, as one aspect of the copolymer of the present invention, the copolymer may be prepared by using a diglycerin fatty acid ester represented by the general formula (1) in which X¹ and X² each represent a hydrogen atom, X³ represents -C(=O)R², and R¹ and R² each represent an isostearyl group.

The diglycerin fatty acid ester to be used in the production of the copolymer of the present invention is not particularly limited as long as the diglycerin fatty acid ester is a diglycerin fatty acid ester represented by the following general formula (1). The diglycerin fatty acid ester may be a diglycerin fatty acid ester derived from petroleum or a diglycerin fatty acid ester derived from a natural raw material. An example of the diglycerin fatty acid ester derived from a natural raw material is a diglycerin fatty acid ester derived from a plant raw material produced by using a specific glycerin fatty acid ester obtained by any one or both of extraction and purification of a plant oil. In the present invention, the copolymer is preferably produced by using the plant raw material-derived diglycerin fatty acid ester represented by the general formula (1) because a copolymer having a high natural origin content can be produced. In the present invention, specifically, a copolymer having a natural origin content of 50% or more is preferably produced, a copolymer having a natural origin content of 70% or more is more preferably produced, and a copolymer having a natural origin content of 80% or more is still more preferably produced by using the plant raw material-derived diglycerin fatty acid ester represented by the general formula (1) as a naturally derived raw material. In the present invention, the natural origin content of the copolymer is a value calculated in conformity with ISO 16128.

The copolymer of the present invention is a copolymer, which is obtained by causing the above-mentioned diglycerin fatty acid ester and isophorone diisocyanate to react with each other, and has a weight-average molecular weight of from 3,500 to 100,000. The weight-average molecular weight of the copolymer is preferably from 4,000 to 85,000, more preferably from 5,000 to 40,000, still more preferably from 5,500 to 25,000, particularly preferably from 6,000 to 21,000 from the viewpoints of the applicability, glossy feeling, and usability of the cosmetic composition to be obtained. In the present invention, the weight-average molecular weight of the copolymer may be adjusted by adjusting the ratio and reaction conditions (e.g., temperature, pressure, time, catalyst, and additive) of the raw material to be used when the diglycerin fatty acid ester and isophorone diisocyanate are caused to react with each other. In addition, in the present invention, the weight-average molecular weight of the copolymer is calculated in terms of styrene through measurement by gel permeation chromatography (GPC).

Measurement conditions for the GPC are as described below.

### [GPC Measurement Conditions]

| | |
|---|---|
| Apparatus: | HLC-8220GPC (manufactured by Tosoh Corporation) |
| Column: | TSKgel SuperMultiporeHZ-N (manufactured by Tosoh Corporation) |
| Column temperature: | 40°C |
| Sample concentration: | 0.5 mass% |
| Developing solvent: | Tetrahydrofuran |
| Detector: | RI detector |
| Reference substance: | TSKgel standard polystyrene (manufactured by Tosoh Corporation) |

The copolymer of the present invention comprises a structure in which a -OH structure portion of the above-mentioned diglycerin fatty acid ester and an isocyanate group of isophorone diisocyanate react with each other. However, one or two or more -OH structure portions in the diglycerin fatty acid ester and the isocyanate group of isophorone diisocyanate arbitrarily react with each other to form a urethane bond, and hence the structure largely differs depending on the specific structure of the diglycerin fatty acid ester to be used and the ratio of the respective raw materials to be used. Accordingly, it is impossible or utterly impractical to unambiguously describe the structure of the copolymer of the present invention by a general formula.

Examples of a method of producing the copolymer of the present invention include: a method comprising causing the diglycerin fatty acid ester and isophorone diisocyanate to react with each other until no reactive isocyanate group is present; and a method comprising causing the diglycerin fatty acid ester and isophorone diisocyanate to react with each other to produce a prepolymer, and then causing the prepolymer to react with a chain extender. Although the kind of the chain extender when the chain extender is used is not particularly limited, for example, one or more kinds selected from the group consisting of: water; ethylenediamine; and propylenediamine may be used.

A solvent may be used in the method of producing the copolymer of the present invention as required. Examples of the solvent include ethanol, propanol, butanol, hexane, toluene, xylene, methyl ethyl ketone, ethyl acetate, butyl acetate, and water.

In the method of producing the copolymer of the present invention, a catalyst may be used for accelerating the reaction between the diglycerin fatty acid ester and isophorone diisocyanate. Examples of the catalyst include: strong acids, such as sulfuric acid and toluenesulfonic acid; metal halides, such as titanium tetrachloride, hafnium chloride, zirconium chloride, aluminum chloride, gallium chloride, indium chloride, iron chloride, tin chloride, and boron fluoride; hydroxides, alcoholates, and carbonates of alkali metals and alkaline earth metals, such as sodium hydroxide, potassium hydroxide, sodium methylate, sodium ethylate, and sodium carbonate; metal oxides, such as aluminum oxide, calcium oxide, barium oxide, and sodium oxide; organic metal compounds, such as tetraisopropyl titanate, dibutyltin dichloride, dibutyltin oxide, and dibutyltin bis(2-ethylhexylthioglycolate); and soaps, such as sodium acetate, potassium acetate, sodium propionate, potassium propionate, sodium octylate, potassium octylate, sodium laurate, and potassium laurate. Although the blending amount of such catalyst is not particularly limited, the amount may be from about 0.001 mass% to about 1 mass% with respect to the total mass of the diglycerin fatty acid ester and isophorone diisocyanate to be used.

In the method of producing the copolymer of the present invention, any other raw material that can react with the diglycerin fatty acid ester or isophorone diisocyanate may be used to the extent that the effects of the present invention are not impaired. However, any one of the following production methods is preferably adopted from the viewpoints of the effects of the present invention: a method in which raw materials consisting of the diglycerin fatty acid ester and isophorone diisocyanate are used, and are caused to react with each other until no reactive isocyanate group is present; and a method comprising causing a reaction using reaction raw materials consisting of the diglycerin fatty acid ester and isophorone diisocyanate to produce a prepolymer, and then causing the prepolymer to react with a chain extender.

The "reaction raw material" as used herein refers to a raw material to be used in a polymerization reaction of the method of producing the copolymer of the present invention.

The method of producing the copolymer of the present invention is not particularly limited as long as the reaction is performed under such a condition that the diglycerin fatty acid ester and isophorone diisocyanate react with each other. In addition, the respective raw materials may be caused to react with each other after their total amount has been collectively loaded, or the raw materials may be caused to react with each other while being loaded in several portions. The following method is given as an example: the respective reaction raw materials containing the diglycerin fatty acid ester and isophorone diisocyanate are loaded into a reaction system in one stroke or in several portions, and are mixed at a temperature of from 30°C to 160°C, preferably from 60°C to 160°C under a pressurized, decompressed, or normal-pressure environment, followed by the maintenance of the mixture for from 30 minutes to 10 hours until a reaction between the raw materials is completed.

Although a field in which the copolymer of the present invention may be used is not particularly limited, for example, the copolymer may be used in various products, such as a coating material, an ink, a pressure-sensitive adhesive or an adhesive, a fuel, a lubricant, and a cosmetic preparation. At this time, a publicly known material may be incorporated into the product, such as a coating material, a pressure-sensitive adhesive or an adhesive, a fuel, a lubricant, or a cosmetic preparation, in accordance with its usage mode and purposes. The copolymer of the present invention is preferably used as a raw material of a cosmetic preparation out of those products because the copolymer of the present invention is excellent in, for example, applicability or adhesive feeling.

When the copolymer of the present invention is used for the cosmetic preparation, the kind of the cosmetic preparation is not particularly limited, and examples thereof include a toner, a serum, a milky lotion, a cream, a face-washing foam, a cleansing milk, a cleansing lotion, a skin mist (a mist toner), a hair tonic, a hair styling liquid, a setting lotion, a hair bleach, a color rinse, a permanent wave solution, a mascara, a lipstick, a lip gloss, a pack, a foundation, Eau de Cologne, a shampoo, a rinse, a hair treatment, a hair wax, a hair oil, a hair milk, a hair manicure, an eyeliner, a sunscreen, a deodorant, a fragrance, a cleansing oil, and a cosmetic oil.

When the copolymer of the present invention is used for the cosmetic preparation, the blending amount of the copolymer in the cosmetic preparation is not particularly limited, and may be adjusted in accordance with, for example, the kind of the cosmetic preparation in which the copolymer is blended and purposes. However, for example, the blending amount may be from 0.01 mass% to 90 mass% with respect to the total amount of the cosmetic preparation. At this time, when the copolymer of the present invention is blended for the purpose of imparting, in particular, for example, applicability, a glossy feeling, and usability to the cosmetic preparation, the copolymer is preferably blended at from 0.01 mass% to 25 mass%, more preferably blended at from 0.05 mass% to 20 mass%, still more preferably blended at from 0.1 mass% to 15 mass%, particularly preferably blended at from 0.5 mass% to 10 mass% with respect to the total amount of the cosmetic preparation. Such cosmetic preparation is not particularly limited, and examples thereof include a toner, a serum, a milky lotion, a cream, a face-washing foam, a cleansing milk, a cleansing lotion, a skin mist (a mist toner), a hair tonic, a hair styling liquid, a setting lotion, a hair bleach, a color rinse, a permanent wave solution, a pack, a foundation, Eau de Cologne, a shampoo, a rinse, a treatment, a hair wax, a hair oil, a hair milk, a hair manicure, an eyeliner, a sunscreen, a deodorant, a fragrance, a cleansing oil, and a cosmetic oil. In addition, when the copolymer of the present invention is blended for the purpose of imparting, in particular, an adhesive feeling to the cosmetic preparation, the copolymer is preferably blended at from 5 mass% to 90 mass%, more preferably blended at from 10 mass% to 85 mass%, still more preferably blended at from 20 mass% to 80 mass%, particularly preferably blended at from 25 mass% to 80 mass% with respect to the total amount of the cosmetic preparation. Such cosmetic preparation is not particularly limited, and examples thereof include a mascara, a lipstick, and a lip gloss.

In addition, a method of adding the copolymer of the present invention to the cosmetic preparation is not particularly limited, and a publicly known method may be used. An example thereof is a method comprising adding the copolymer of the present invention to a blend obtained by mixing part or all of the other components of the cosmetic preparation under normal temperature or a temperature-controlled environment, followed by stirring or the like as required. The copolymer of the present invention has the above-mentioned structure, and hence, when the copolymer is added to the cosmetic preparation, various characteristics thereof can be improved at the same time.

The cosmetic composition of the present invention is a cosmetic composition containing the above-mentioned copolymer. Examples of such cosmetic composition include a toner, a serum, a milky lotion, a cream, a face-washing foam, a cleansing milk, a cleansing lotion, a skin mist (a mist toner), a hair tonic, a hair styling liquid, a setting lotion, a hair bleach, a color rinse, a permanent wave solution, a mascara, a lipstick, a lip gloss, a pack, a foundation, Eau de Cologne, a shampoo, a rinse, a hair treatment, a hair wax, a hair oil, a hair milk, a hair manicure, an eyeliner, a sunscreen, a deodorant, a fragrance, a cleansing oil, and a cosmetic oil. The properties of the cosmetic preparation of the present invention may be appropriately adjusted in accordance with its use purposes or product form, and the cosmetic preparation may be, for example, a liquid form, an emulsion form, a gel form, a cream form, solid powder, a foam form, or a spray form.

The content of the above-mentioned copolymer in the cosmetic composition of the present invention is not particularly limited, and may be adjusted in accordance with its purposes or applications. The content may be, for example, from 0.01 mass% to 90 mass% with respect to the total amount of the cosmetic composition. At this time, when the cosmetic composition contains the copolymer of the present invention for the purpose of imparting, in particular, for example, applicability, a glossy feeling, and usability to the cosmetic preparation, the content of the copolymer in the cosmetic composition is preferably from 0.01 mass% to 25 mass%, more preferably from 0.05 mass% to 20 mass%, still more preferably from 0.1 mass% to 15 mass%, particularly preferably from 0.5 mass% to 10 mass% with respect to the total amount of the cosmetic composition. At this time, examples of the cosmetic composition include a toner, a serum, a milky lotion, a cream, a face-washing foam, a cleansing milk, a cleansing lotion, a skin mist, a hair tonic, a hair styling liquid, a setting lotion, a hair bleach, a color rinse, a permanent wave solution, a pack, a foundation, Eau de Cologne, a shampoo, a rinse, a hair treatment, a hair wax, a hair oil, a hair milk, a hair manicure, an eyeliner, a sunscreen, a deodorant, a fragrance, a cleansing oil, and a cosmetic oil. In addition, when the cosmetic composition contains the copolymer of the present invention for the purpose of imparting, in particular, an adhesive feeling to the cosmetic preparation, the content of the copolymer in the cosmetic composition is preferably from 5 mass% to 90 mass%, more preferably from 10 mass% to 85 mass%, still more preferably from 20 mass% to 80 mass%, particularly preferably from 25 mass% to 80 mass% with respect to the total amount of the cosmetic composition. At this time, examples of the cosmetic composition include a mascara, a lipstick, and a lip gloss.

In addition to the copolymer described above, any component to be generally used in a cosmetic composition for improving and modifying various characteristics (e.g., solubility, dispersibility, stability, a use feeling, applicability, permeability, moisture retentivity, safety, a designability, an optical characteristic, aromaticity, and a whitening property) at the time of its storage, at the time of its use, and after the use in accordance with its usage mode or use purposes may be used in the cosmetic composition of the present invention. Examples of such component include a cationic surfactant, an anionic surfactant, an amphoteric surfactant, a non-ionic surfactant, a hydrocarbon oil, a silicone oil, an ester oil, a higher alcohol, a polyhydric alcohol, a sugar and a derivative thereof, a pH-adjusting agent, a dye or a pigment, a perfume, a UV absorber, and a solvent. Those additives may be arbitrarily blended alone or in combination thereof.

Examples of the cationic surfactant include lauryltrimethylammonium chloride, cetyltrimethylammonium chloride, stearyltrimethylammonium chloride, an alkyltrimethylammonium chloride, distearyldimethylammonium chloride, stearyltrimethylammonium saccharin, cetyltrimethylammonium saccharin, behenyltrimethylammonium methylsulfate, behenyldimethylamine, diethylaminoethylamide behenate, dimethylaminopropylamide behenate, dimethylaminoethylamide behenate, stearyldimethylamine, palmitoxypropyldimethylamine, and stearoxypropyldimethylamine. Those cationic surfactants may be used alone or in combination thereof. The concentration of the cationic surfactant may be set to, for example, from 0.001 mass% to 10 mass% with respect to the total amount of the cosmetic composition, and is preferably from 0.01 mass% to 5 mass%.

Examples of the anionic surfactant include an alkyl ether sulfuric acid salt, an alkyl sulfuric acid salt, an alkyl ether sulfuric acid ester salt, an alkenyl ether sulfuric acid salt, an alkenyl sulfuric acid salt, an olefin sulfonic acid salt, an alkane sulfonic acid salt, a saturated or unsaturated fatty acid salt, an alkyl or alkenyl ether carboxylic acid salt, an α-sulfone fatty acid salt, a N-acyl amino acid-type surfactant, a phosphoric acid mono or diester-type surfactant, a sulfosuccinic acid ester, a N-alkyloyl methyl taurine salt, and derivatives thereof. In addition, as a counterion of the anionic group, there are specifically given, for example, a sodium ion, a potassium ion, and triethanolamine. Those counterions may be used alone or in combination thereof. The concentration of the anionic surfactant may be set to, for example, from 0.001 mass% to 10 mass% with respect to the total amount of the cosmetic composition, and is preferably from 0.01 mass% to 5 mass%.

Examples of the amphoteric surfactant include: betaine-type amphoteric surfactants, such as a cocamidopropyl betaine, lauryldimethylaminoacetic acid betaine, a 2-alkyl-N-carboxymethyl-N-hydroxymethyl imidazolinium betaine, lauryl hydroxy sulfobetaine, lauroyl amide ethyl hydroxyethyl carboxymethyl betaine, and a metal salt of hydroxypropyl phosphoric acid; amino acid-type amphoteric surfactants such as a metal salt of β-laurylamino propionic acid; and a sulfuric acid ester-type amphoteric surfactant and a sulfonic acid-type amphoteric surfactant. Those amphoteric surfactants may be used alone or in combination thereof. The concentration of the amphoteric surfactant may be set to, for example, from 0.001 mass% to 10 mass% with respect to the total amount of the cosmetic composition, and is preferably from 0.01 mass% to 5 mass%.

Examples of the non-ionic surfactant include POE-cetyl ether (ceteth), POE-stearyl ether (steareth), POE-behenyl ether, POE-oleyl ether (oleth), POE-lauryl ether (laureth), POE-octyldodecyl ether, POE-hexyldecyl ether, POE-isostearyl ether, POE-nonylphenyl ether, POE-octylphenyl ether, POE-polyoxypropylene cetyl ether, POE-polyoxypropylene decyl tetradecyl ether, POE-sorbitan monooleate, POE-sorbitan monostearate, POE-sorbitan monopalmitate, POE-sorbitan monolaurate, POE-sorbitan trioleate, POE-glycerin monostearate, POE-glycerin monomyristate, POE-sorbit tetraoleate, POE-sorbit hexastearate, POE-sorbit monolaurate, POE-sorbit beeswax, polyethylene glycol monooleate, polyethylene glycol monostearate, polyethylene glycol monolaurate, lipophilic glycerin monooleate, lipophilic glycerin monostearate, self-emulsifiable glycerin monostearate, sorbitan monooleate, sorbitan sesquioleate, sorbitan trioleate, sorbitan monostearate, sorbitan monopalmitate, sorbitan monolaurate, a sucrose fatty acid ester, decaglyceryl monolaurate, decaglyceryl monostearate, decaglyceryl monooleate, decaglyceryl monomyristate, an alkyl glucoside, POE-methyl glucoside, and POE-methyl glucoside dioleate. Those non-ionic surfactants may be used alone or in combination thereof. The concentration of the non-ionic surfactant may be set to, for example, from 0.001 mass% to 10 mass% with respect to the total amount of the cosmetic composition, and is preferably from 0.01 mass% to 5 mass%.

Examples of the hydrocarbon oil include liquid paraffin, squalene, pristane, ozocerite, paraffin, ceresin, Vaseline, polyisobutene, polyisoprene, isodecane, isododecane, isohexadecane, normal pentane, isopentane, normal hexane, isohexane, kerosine, decalin, tetralin, and microcrystalline wax. Those hydrocarbon oils may be used alone or in combination thereof. The concentration of the hydrocarbon oil may be set to, for example, from 0.1 mass% to 50 mass% with respect to the total amount of the cosmetic composition, and is preferably from 0.5 mass% to 30 mass%.

Examples of the silicone oil include: chain silicone oils, such as dimethylpolysiloxane, dimethiconol, diphenylpolysiloxane, diphenylsiloxy phenyl trimethicone, and octamethyltrisiloxane; cyclic silicone oils, such as decamethylcyclotetrasiloxane, dodecamethylcyclotetrasiloxane, octamethylcyclotetrasiloxane, cyclopentasiloxane, dodecamethylcyclopentasiloxane, octamethylcyclopentasiloxane, decamethylcyclohexasiloxane, dodecamethylcyclohexasiloxane, and octamethylcyclohexasiloxane; and modified silicone oils, such as an alkyl-modified dimethylpolysiloxane, a polyether-modified dimethylpolysiloxane, a carbinol-modified polysiloxane, a fatty acid-modified polysiloxane, a higher alcohol-modified polysiloxane, an amino-modified polysiloxane, and a fluorine-modified polysiloxane. Those silicone oils may be used alone or in combination thereof. The concentration of the silicone oil may be set to, for example, from 0.1 mass% to 50 mass% with respect to the total amount of the cosmetic composition, and is preferably from 0.5 mass% to 30 mass%.

Examples of the ester oil include: synthetic ester oils, such as ethyl acetate, butyl acetate, hexyl acetate, decyl acetate, butyl propionate, cetyl octanoate, hexyldecyl dimethyloctanoate, isononyl isononanoate, isononyl isononanoate, isotridecyl isononanoate, ethyl laurate, hexyl laurate, myristyl myristate, isopropyl myristate, isopropyl palmitate, 2-ethylhexyl palmitate, 2-hexyldecyl palmitate, 2-heptylundecyl palmitate, decyl oleate, oleyl oleate, octyldodecyl oleate, isocetyl stearate, glycerin stearate, butyl stearate, ethylhexyl hydroxystearate, ethylene glycol stearate, octyl oxystearate, diethyl phthalate, triethyl citrate, 2-ethylhexyl succinate, diisopropyl sebacate, di-2-ethylhexyl sebacate, cetyl lactate, myristyl lactate, diisobutyl adipate, 2-hexyldecyl adipate, di-2-heptylundecyl adipate, cetyl ethylhexanoate, triethylhexanoin, polyglyceryl-2 triisostearate, neopentyl glycol dioctanoate, neopentyl glycol dicaprate, and pentaerythrityl tetraisostearate; and animal and plant ester oils, such as lanolin, mink oil, cacao butter, coconut oil, palm kernel oil, camellia oil, sesame oil, castor oil, and olive oil. Those ester oils may be used alone or in combination thereof. The concentration of the ester oil may be set to, for example, from 0.1 mass% to 50 mass% with respect to the total amount of the cosmetic composition, and is preferably from 0.5 mass% to 30 mass%.

Examples of the higher alcohol include cetyl alcohol, isostearyl alcohol, lauryl alcohol, hexadecyl alcohol, and octyl dodecanol. Those higher alcohols may be used alone or in combination thereof. The concentration of the higher alcohol may be set to, for example, from 0.1 mass% to 30 mass% with respect to the total amount of the cosmetic composition, and is preferably from 0.5 mass% to 20 mass%.

Examples of the polyhydric alcohol include ethylene glycol, diethylene glycol, triethylene glycol, polyethylene glycol, highly polymerized polyethylene glycol, propylene glycol, dipropylene glycol, polypropylene glycol, isoprene glycol, 1,3-butylene glycol, glycerin, diglycerin, and polyglycerin. Those polyhydric alcohols may be used alone or in combination thereof. The concentration of the polyhydric alcohol may be set to, for example, from 0.1 mass% to 30 mass% with respect to the total amount of the cosmetic composition, and is preferably from 0.5 mass% to 20 mass%.

Examples of the sugar and the derivative thereof include xylose, D-glucose, sucrose, trehalose, fructose, maltose, mannose, cyclodextrin, β-glucan, chitin, chitosan, pectin, arabinogalactan, dextrin, dextran, and an ethyl glucosyl methacrylate polymer or copolymer. Those sugars and the derivatives thereof may be used alone or in combination thereof. The concentration of the sugar and the derivative thereof may be set to, for example, from 0.001 mass% to 10 mass% with respect to the total amount of the cosmetic composition, and is preferably from 0.01 mass% to 5 mass%.

Examples of the pH-adjusting agent include citric acid, glycolic acid, succinic acid, tartaric acid, lactic acid, malic acid, levulinic acid, acetic acid, butyric acid, valeric acid, oxalic acid, maleic acid, fumaric acid, mandelic acid, phosphoric acid, pyrophosphoric acid, hydrochloric acid, sulfuric acid, and nitric acid. Those pH-adjusting agents may be used alone or in combination thereof. The pH-adjusting agent is preferably added so that the pH of, for example, the cosmetic composition of the present invention may be from 3.0 to 13.0.

Examples of the dye or the pigment include various certified colors, an acidic dye, a basic dye, an oxidation dye intermediate, a coupler, an autoxidation-type dye, a nitro dye, a dispersion dye, an inorganic pigment, and a metal powder pigment, and surface-treated products thereof. Those dyes or pigments may be used alone or in combination thereof. The concentration of the dye or the pigment may be set to, for example, from 0.001 mass% to 10 mass% with respect to the total amount of the cosmetic composition, and is preferably from 0.01 mass% to 5 mass%.

Examples of the perfume include acetylcedrene, allyl amyl glycolate, β-ionone, isobutyl quinoline, orris oil, irone, indole, undecanal, undecenal, γ-undecalactone, estragole, eugenol, oakmoss, opoponax resinoid, orange oil, aurantiol, galaxolide, carvacrol, camphor, carrot seed oil, clove oil, methyl cinnamate, geraniol, geranyl nitrile, isobornyl acetate, geranyl acetate, dimethyl benzyl carbinyl acetate, styrallyl acetate, cedryl acetate, terpinyl acetate, vetiveryl acetate, benzyl acetate, linalyl acetate, isopentyl salicylate, benzyl salicylate, sandalwood oil, santalol, cyclamen aldehyde, cyclopentadecanolide, dihydrojasmonic acid methyl, dihydromyrcenol, jasmine absolute, jasmine lactone, citral, citronellol, citronellal, cinnamon bark oil, styrax resinoid, cedarwood oil, cedrene, cedrol, celery seed oil, thyme oil, damascone, damascenone, thymol, tuberose absolute, terpineol, γ-terpinene, triplal, vanilla absolute, vanillin, basil oil, patchouli oil, hydroxy citronellal, α-pinene, piperitone, peru balsam, vetiver oil, vetiverol, peppermint oil, pepper oil, heliotropine, bergamot oil, benzyl benzoate, borneol, myrrh resinoid, musk ketone, methyl nonyl acetoaldehyde, γ-methyl ionone, menthol, L-menthol, L-menthone, eucalyptus oil, lime oil, lavender oil, D-limonene, linalool, lyral, lilial, lemon oil, rose absolute, rose oxide, rose oil, and rosemary oil. Those perfumes may be used alone or in combination thereof. The concentration of the perfume may be set to, for example, from 0.001 mass% to 5 mass% with respect to the total amount of the cosmetic composition, and is preferably from 0.01 mass% to 3 mass%.

Examples of the UV absorber include 2,4-dihydroxybenzophenone, 5,5'-methylenebis(2-hydroxy-4-methoxybenzophenone), 2-(2-hydroxy-5-methylphenyl)benzotriazole, 2-(2-hydroxy-5-tert-octylphenyl)benzotriazole, 2-(2-hydroxy-3,5-di-tert-butylphenyl)-5-chlorobenzotriazole, 2-(2-hydroxy-3-tert-butyl-5-methylphenyl)-5-chlorobenzotriazole, 2-(2-hydroxy-3,5-dicumylphenyl)benzotriazole, 2,2'-methylenebis(4-tert-octyl-6-benzotriazolylphenol), a polyethylene glycol ester of 2-(2-hydroxy-3-tert-butyl-5-carboxy phenyl)benzotriazole, 2-[2-hydroxy-3-(2-acryloyloxyethyl)-5-methylphenyl]benzotriazole, 2-[2-hydroxy-3-(2-methacryloyloxyethyl)-5-tert-butylphenyl]benzotriazole, 2-[2-hydroxy-3-(2-methacryloyloxyethyl)-5-tert-octylphenyl]benzotriazole, 2-[2-hydroxy-3-(2-methacryloyloxyethyl)-5-tert-butylphenyl]-5-chlorobenzotriazole, 2-[2-hydroxy-5-(2-methacryloyloxyethyl)phenyl]benzotriazole, 2-[2-hydroxy-3-tert-butyl-5-(2-methacryloyloxyethyl)phenyl]benzotriazole, 2-[2-hydroxy-3-tert-amyl-5-(2-methacryloyloxyethyl)phenyl]benzotriazole, 2-[2-hydroxy-3-tert-butyl-5-(3-methacryloyloxypropyl)phenyl]-5-chlorobenzotriazole, 2-[2-hydroxy-4-(2-methacryloyloxymethyl)phenyl]benzotriazole, 2-[2-hydroxy-4-(3-methacryloyloxy-2-hydroxypropyl)phenyl]benzotriazole, 2-[2-hydroxy-4-(3-methacryloyloxypropyl)phenyl]benzotriazole, phenyl salicylate, resorcinol monobenzoate, 2,4-di-tert-butylphenyl-3,5-di-tert-butyl-4-hydroxybenzoate, octyl (3,5-di-tert-butyl-4-hydroxy)benzoate, dodecyl (3,5-di-tert-butyl-4-hydroxy)benzoate, tetradecyl (3,5-di-tert-butyl-4-hydroxy)benzoate, hexadecyl (3,5-di-tert-butyl-4-hydroxy)benzoate, octadecyl (3,5-di-tert-butyl-4-hydroxy)benzoate, behenyl (3,5-di-tert-butyl-4-hydroxy)benzoate, 2-ethyl-2'-ethoxyoxanilide, 2-ethoxy-4'-dodecyloxanilide, ethyl-α-cyano-β,β-diphenyl acrylate, methyl-2-cyano-3-methyl-3-(p-methoxyphenyl) acrylate, ethylhexyl methoxycinnamate, bisethylhexyloxyphenol methoxyphenyl triazine, diethylamino hydroxybenzoyl hexyl benzoate, and various metal salts or metal chelates. Those UV absorbers may be used alone or in combination thereof. The concentration of the UV absorber may be set to, for example, from 0.001 mass% to 10 mass% with respect to the total amount of the cosmetic composition, and is preferably from 0.01 mass% to 5 mass%.

Examples of the solvent include ethanol, isopropyl alcohol, butanol, isobutyl alcohol, acetone, ethyl acetate, ethylene glycol monoethyl ether, and water. Those solvents may be used alone or in combination thereof. The concentration of the solvent may be set to, for example, from 10 mass% to 99 mass% with respect to the total amount of the cosmetic composition, and is preferably from 20 mass% to 95 mass%.

The viscosity of the cosmetic composition of the present invention at 25°C is not particularly limited, and may be appropriately adjusted in accordance with its use purposes and product form. However, for example, the viscosity of the cosmetic composition at 25°C is preferably from 10 mPa·s to 1,000,000 mPa·s, more preferably from 100 mPa·s to 100,000 mPa·s from the viewpoint of utilizing the applicability, adhesive feeling, glossy feeling, and usability of the above-mentioned copolymer. In the present invention, the viscosity of the cosmetic composition at 25°C is measured in conformity with JIS K 7117.

### Examples

Details about the present invention are described below by way of the Examples and the Comparative Examples, but the present invention is not limited by these examples. In Examples and the like below, the symbol "%" is on a mass basis unless otherwise stated.

### <Example 1>

450 Grams (0.60 mol) of polyglyceryl-2 diisostearate (diglycerin fatty acid ester represented by the general formula (1) in which R¹ represented an isostearyl group, X¹ and X² each represented a hydrogen atom, X³ represented a group represented by -C(=O)R², and R² represented an isostearyl group) derived from a plant raw material and 60.3 g (0.27 mol) of isophorone diisocyanate were loaded into a glass-made reaction vessel comprising a stirring machine, a cooling tube, and a nitrogen-introducing tube, and were caused to react with each other at 120°C for 1 hour. The fact that no NCO absorption was present was recognized by the infrared absorption spectrum of the reaction product, and then the reaction was terminated. Thus, a copolymer 1 consisting of a (polyglyceryl-2 diisostearate/IPDI) copolymer was produced. The weight-average molecular weight of the resultant copolymer 1 determined in terms of styrene through measurement by gel permeation chromatography (GPC) was 4,300.

### <Example 2>

450 Grams (0.60 mol) of polyglyceryl-2 diisostearate derived from a plant raw material and 67 g (0.30 mol) of isophorone diisocyanate were loaded into a glass-made reaction vessel comprising a stirring machine, a cooling tube, and a nitrogen-introducing tube, and were caused to react with each other at 120°C for 1 hour. The fact that no NCO absorption was present was recognized by the infrared absorption spectrum of the reaction product, and then the reaction was terminated. Thus, a copolymer 2 consisting of a (polyglyceryl-2 diisostearate/IPDI) copolymer was produced. The weight-average molecular weight of the resultant copolymer 2 determined in terms of styrene through measurement by gel permeation chromatography (GPC) was 5,100.

### <Example 3>

450 Grams (0.60 mol) of polyglyceryl-2 diisostearate derived from a plant raw material and 73.7 g (0. 33 mol) of isophorone diisocyanate were loaded into a glass-made reaction vessel comprising a stirring machine, a cooling tube, and a nitrogen-introducing tube, and were caused to react with each other at 120°C for 1 hour. The fact that no NCO absorption was present was recognized by the infrared absorption spectrum of the reaction product, and then the reaction was terminated. Thus, a copolymer 3 consisting of a (polyglyceryl-2 diisostearate/IPDI) copolymer was produced. The weight-average molecular weight of the resultant copolymer 3 determined in terms of styrene through measurement by gel permeation chromatography (GPC) was 6,400.

### <Example 4>

450 Grams (0.60 mol) of polyglyceryl-2 diisostearate derived from a plant raw material and 86.9 g (0.39 mol) of isophorone diisocyanate were loaded into a glass-made reaction vessel comprising a stirring machine, a cooling tube, and a nitrogen-introducing tube, and were caused to react with each other at 120°C for 1 hour. The fact that no NCO absorption was present was recognized by the infrared absorption spectrum of the reaction product, and then the reaction was terminated. Thus, a copolymer 4 consisting of a (polyglyceryl-2 diisostearate/IPDI) copolymer was produced. The weight-average molecular weight of the resultant copolymer 4 determined in terms of styrene through measurement by gel permeation chromatography (GPC) was 9,200.

### <Example 5>

450 Grams (0.60 mol) of polyglyceryl-2 diisostearate derived from a plant raw material and 100.3 g (0.45 mol) of isophorone diisocyanate were loaded into a glass-made reaction vessel comprising a stirring machine, a cooling tube, and a nitrogen-introducing tube, and were caused to react with each other at 120°C for 1 hour. The fact that no NCO absorption was present was recognized by the infrared absorption spectrum of the reaction product, and then the reaction was terminated. Thus, a copolymer 5 consisting of a (polyglyceryl-2 diisostearate/IPDI) copolymer was produced. The weight-average molecular weight of the resultant copolymer 5 determined in terms of styrene through measurement by gel permeation chromatography (GPC) was 20,000.

### <Example 6>

450 Grams (0.60 mol) of polyglyceryl-2 diisostearate derived from a plant raw material and 113.6 g (0.51 mol) of isophorone diisocyanate were loaded into a glass-made reaction vessel comprising a stirring machine, a cooling tube, and a nitrogen-introducing tube, and were caused to react with each other at 120°C for 1 hour. The fact that no NCO absorption was present was recognized by the infrared absorption spectrum of the reaction product, and then the reaction was terminated. Thus, a copolymer 6 consisting of a (polyglyceryl-2 diisostearate/IPDI) copolymer was produced. The weight-average molecular weight of the resultant copolymer 6 determined in terms of styrene through measurement by gel permeation chromatography (GPC) was 83,000.

### <Comparative Example 1>

450 Grams (0.60 mol) of polyglyceryl-2 diisostearate derived from a plant raw material and 46.8 g (0.21 mol) of isophorone diisocyanate were loaded into a glass-made reaction vessel comprising a stirring machine, a cooling tube, and a nitrogen-introducing tube, and were caused to react with each other at 120°C for 1 hour. The fact that no NCO absorption was present was recognized by the infrared absorption spectrum of the reaction product, and then the reaction was terminated. Thus, a copolymer 7 consisting of a (polyglyceryl-2 diisostearate/IPDI) copolymer was produced. The weight-average molecular weight of the resultant copolymer 7 determined in terms of styrene through measurement by gel permeation chromatography (GPC) was 2,900.

### <Comparative Example 2>

450 Grams (0.60 mol) of polyglyceryl-2 diisostearate derived from a plant raw material and 116.3 g (0.52 mol) of isophorone diisocyanate were loaded into a glass-made reaction vessel comprising a stirring machine, a cooling tube, and a nitrogen-introducing tube, and were caused to react with each other at 120°C for 1 hour. The fact that no NCO absorption was present was recognized by the infrared absorption spectrum of the reaction product, and then the reaction was terminated. Thus, a copolymer 8 consisting of a (polyglyceryl-2 diisostearate/IPDI) copolymer was produced. The weight-average molecular weight of the resultant copolymer 8 determined in terms of styrene through measurement by gel permeation chromatography (GPC) was 130,000.

Applicability, an adhesive feeling, and a glossy feeling on an applied surface at the time of the taking of an appropriate amount of a solution obtained by diluting each of the copolymers obtained in Examples 1 to 6, and Comparative Examples 1 and 2 with liquid paraffin (manufactured by Kaneda Co., Ltd., HICALL K-230) to 50% on a finger and the application thereof to the back of a hand, and usability after the application were individually evaluated by three panelists based on the following evaluation criteria. Evaluation was made as follows: an evaluation "oo" (Excellent) was given when the three panelists evaluated the solution as "o"; an evaluation "o" (Good) was given when two panelists evaluated the solution as "o"; and an evaluation "×" (Poor) was given when less than two panelists evaluated the solution as "o". The respective results of the evaluation are shown in Table 1. In the following evaluation criteria, "o" means a "success", and "×" means a "failure".

### [Evaluation Criteria for Applicability]

o: Lubricity is present, and the solution is easily applied to the skin.
×: Roughness is felt, or the solution is hard and is difficult to apply to the skin.

### [Evaluation Criteria for Adhesive Feeling]

o: Adhesiveness to the skin is excellent, and a sticky feeling is absent.
×: Adhesiveness is weak, or a sticky feeling is present.

### [Evaluation Criteria for Glossy Feeling]

o: An apparent glossy feeling is felt on the applied surface.
×: A glossy feeling is absent on the applied surface, or is substantially unfelt.

### [Evaluation Criteria for Usability]

o: The solution is not washed away with flowing water (15°C), and is easily removed with hot water (40°C).
×: The solution is easily washed away with flowing water (15°C), or not washed away even with hot water (40°C).

**Table 1**

| | Example 1 | Example 2 | Example 3 | Example 4 | Example 5 | Example 6 | Comparative Example 1 | Comparative Example 2 |
|---|---|---|---|---|---|---|---|---|
| | Copolymer 1 | Copolymer 2 | Copolymer 3 | Copolymer 4 | Copolymer 5 | Copolymer 6 | Copolymer 7 | Copolymer 8 |
| Weight-average molecular weight | 4, 300 | 5, 100 | 6, 400 | 9,200 | 20,000 | 83,000 | 2,900 | 130,000 |
| Applicability | ○ | ○ | ○○ | ○○ | ○○ | ○ | × | × |
| Adhesive feeling | ○ | ○ | ○○ | ○○ | ○○ | ○○ | × | × |
| Glossy feeling | ○ | ○○ | ○○ | ○○ | ○○ | ○ | × | × |
| Usability | ○○ | ○○ | ○○ | ○○ | ○○ | ○ | × | × |

As is apparent from the above-mentioned results, it was found that the copolymer of the present invention was excellent in all of applicability, adhesive feeling, glossy feeling, and usability. Accordingly, the copolymer of the present invention may be widely used for the purpose of improving various characteristics of products, such as a coating material, an ink, a pressure-sensitive adhesive or an adhesive, a fuel, a lubricant, and a cosmetic preparation. It is understood that, particularly when the copolymer is blended in a cosmetic preparation, applicability, an adhesive feeling, a glossy feeling, and usability can be imparted to a cosmetic composition.

As cosmetic preparations each containing the copolymer of the present invention, Formulation Examples of cosmetic compositions each containing the copolymers 1 to 6 produced in Examples 1 to 6 are described in the following Tables 2 to 33. The numerical values in the tables represent the contents (mass%) of the respective components in the respective cosmetic compositions.

**Table 2**

| Lip gloss 1 | | | | | | |
|---|---|---|---|---|---|---|
| | Formulation Example 1 | Formulation Example 2 | Formulation Example 3 | Formulation Example 4 | Formulation Example 5 | Formulation Example 6 |
| Triethylhexanoin | 40.00 | 40.00 | 40.00 | 40.00 | 40.00 | 40.00 |
| Dimethylsilylated silica | 5.00 | 5.00 | 5.00 | 5.00 | 5.00 | 5.00 |
| Pentylene glycol | 1.00 | 1.00 | 1.00 | 1.00 | 1.00 | 1.00 |
| Red No. 218 | 0.30 | 0.30 | 0.30 | 0.30 | 0.30 | 0.30 |
| Copolymer 1 | 2.00 | | | | | |
| Copolymer 2 | | 2.00 | | | | |
| Copolymer 3 | | | 2.00 | | | |
| Copolymer 4 | | | | 2.00 | | |
| Copolymer 5 | | | | | 2.00 | |
| Copolymer 6 | | | | | | 2.00 |
| Diisostearyl malate | Balance | Balance | Balance | Balance | Balance | Balance |
| Total | 100 | 100 | 100 | 100 | 100 | 100 |

**Table 3**

| Lip gloss 2 | | | | | | |
|---|---|---|---|---|---|---|
| | Formulation Example 7 | Formulation Example 8 | Formulation Example 9 | Formulation Example 10 | Formulation Example 11 | Formulation Example 12 |
| Ethylhexyl palmitate | 5.00 | 5.00 | 5.00 | 5.00 | 5.00 | 5.00 |
| Hydrogenated polyisobutene | 5.00 | 5.00 | 5.00 | 5.00 | 5.00 | 5.00 |
| Pentylene glycol | 1.00 | 1.00 | 1.00 | 1.00 | 1.00 | 1.00 |
| Copolymer 1 | 80.00 | | | | | |
| Copolymer 2 | | 80.00 | | | | |
| Copolymer 3 | | | 70.00 | | | |
| Copolymer 4 | | | | 70.00 | | |
| Copolymer 5 | | | | | 50.00 | |
| Copolymer 6 | | | | | | 50.00 |
| Diisostearyl malate | Balance | Balance | Balance | Balance | Balance | Balance |
| Total | 100 | 100 | 100 | 100 | 100 | 100 |

**Table 4**

| Lip gloss 3 | | | | | | |
|---|---|---|---|---|---|---|
| | Formulation Example 13 | Formulation Example 14 | Formulation Example 15 | Formulation Example 16 | Formulation Example 17 | Formulation Example 18 |
| PEG-6 sorbitan stearate | 3.00 | 3.00 | 3.00 | 3.00 | 3.00 | 3.00 |
| Methyl trimethicone | 10.00 | 10.00 | 10.00 | 10.00 | 10.00 | 10.00 |
| Paraffin | 10.00 | 10.00 | 10.00 | 10.00 | 10.00 | 10.00 |
| Mica titanium treated with 30 perfluorooctyltriethoxysilane | 5.50 | 5.50 | 5.50 | 5.50 | 5.50 | 5.50 |
| Pentylene glycol | 1.00 | 1.00 | 1.00 | 1.00 | 1.00 | 1.00 |
| Red No. 202 | 0.10 | 0.10 | 0.10 | 0.10 | 0.10 | 0.10 |
| Yellow No. 4 | 0.60 | 0.60 | 0.60 | 0.60 | 0.60 | 0.60 |
| Black iron oxide | 0.10 | 0.10 | 0.10 | 0.10 | 0.10 | 0.10 |
| Red No. 104 | 0.50 | 0.50 | 0.50 | 0.50 | 0.50 | 0.50 |
| Copolymer 1 | 30.00 | | | | | |
| Copolymer 2 | | 30.00 | | | | |
| Copolymer 3 | | | 25.00 | | | |
| Copolymer 4 | | | | 25.00 | | |
| Copolymer 5 | | | | | 20.00 | |
| Copolymer 6 | | | | | | 20.00 |
| Diisostearyl malate | Balance | Balance | Balance | Balance | Balance | Balance |
| Total | 100 | 100 | 100 | 100 | 100 | 100 |

**Table 5**

| Lip cream | | | | | | |
|---|---|---|---|---|---|---|
| | Formulation Example 19 | Formulation Example 20 | Formulation Example 21 | Formulation Example 22 | Formulation Example 23 | Formulation Example 24 |
| Vaseline | 5.00 | 5.00 | 5.00 | 5.00 | 5.00 | 5.00 |
| Candelilla wax | 10.00 | 10.00 | 10.00 | 10.00 | 10.00 | 10.00 |
| Stearic acid | 5.00 | 5.00 | 5.00 | 5.00 | 5.00 | 5.00 |
| Beeswax | 5.00 | 5.00 | 5.00 | 5.00 | 5.00 | 5.00 |
| Squalane | 5.00 | 5.00 | 5.00 | 5.00 | 5.00 | 5.00 |
| Glyceryl tri(caprylate/capr ate) | 20.00 | 20.00 | 20.00 | 20.00 | 20.00 | 20.00 |
| Pentylene glycol | 1.00 | 1.00 | 1.00 | 1.00 | 1.00 | 1.00 |
| Phenoxyethanol | 0.50 | 0.50 | 0.50 | 0.50 | 0.50 | 0.50 |
| Copolymer 1 | 10.00 | | | | | |
| Copolymer 2 | | 10.00 | | | | |
| Copolymer 3 | | | 10.00 | | | |
| Copolymer 4 | | | | 10.00 | | |
| Copolymer 5 | | | | | 10.00 | |
| Copolymer 6 | | | | | | 10.00 |
| Liquid lanolin | Balance | Balance | Balance | Balance | Balance | Balance |
| Total | 100 | 100 | 100 | 100 | 100 | 100 |

**Table 6**

| Lipstick 1 | | | | | | |
|---|---|---|---|---|---|---|
| | Formulation Example 25 | Formulation Example 26 | Formulation Example 27 | Formulation Example 28 | Formulation Example 29 | Formulation Example 30 |
| Squalane | 10.00 | 10.00 | 10.00 | 10.00 | 10.00 | 10.00 |
| Isononyl isononanoate | 8.00 | 8.00 | 8.00 | 8.00 | 8.00 | 8.00 |
| Microcrystalline wax | 12.00 | 12.00 | 12.00 | 12.00 | 12.00 | 12.00 |
| Polyethylene | 4.00 | 4.00 | 4.00 | 4.00 | 4.00 | 4.00 |
| Methylparaben | 0.20 | 0.20 | 0.20 | 0.20 | 0.20 | 0.20 |
| BHT | 0.05 | 0.05 | 0.05 | 0.05 | 0.05 | 0.05 |
| Perfume | 0.50 | 0.50 | 0.50 | 0.50 | 0.50 | 0.50 |
| Red No. 202 | 0.10 | 0.10 | 0.10 | 0.10 | 0.10 | 0.10 |
| Yellow No. 4 | 0.50 | 0.50 | 0.50 | 0.50 | 0.50 | 0.50 |
| Iron oxide | 0.10 | 0.10 | 0.10 | 0.10 | 0.10 | 0.10 |
| Titanium oxide | 1.00 | 1.00 | 1.00 | 1.00 | 1.00 | 1.00 |
| Copolymer 1 | 10.00 | | | | | |
| Copolymer 2 | | 10.00 | | | | |
| Copolymer 3 | | | 10.00 | | | |
| Copolymer 4 | | | | 10.00 | | |
| Copolymer 5 | | | | | 10.00 | |
| Copolymer 6 | | | | | | 10.00 |
| Diisostearyl malate | Balance | Balance | Balance | Balance | Balance | Balance |
| Total | 100 | 100 | 100 | 100 | 100 | 100 |

**Table 7**

| Lipstick 2 | | | | | | |
|---|---|---|---|---|---|---|
| | Formulation Example 31 | Formulation Example 32 | Formulation Example 33 | Formulation Example 34 | Formulation Example 35 | Formulation Example 36 |
| Polyethylene | 8.00 | 8.00 | 8.00 | 8.00 | 8.00 | 8.00 |
| Vaseline | 3.00 | 3.00 | 3.00 | 3.00 | 3.00 | 3.00 |
| Cyclopentasiloxane | 22.00 | 22.00 | 22.00 | 22.00 | 22.00 | 22.00 |
| PG dicaprate | 5.00 | 5.00 | 5.00 | 5.00 | 5.00 | 5.00 |
| PEG-6 sorbitan distearate | 1.00 | 1.00 | 1.00 | 1.00 | 1.00 | 1.00 |
| Iron oxide mica titanium | 0.10 | 0.10 | 0.10 | 0.10 | 0.10 | 0.10 |
| Sorbitan sesquioleate | 1.00 | 1.00 | 1.00 | 1.00 | 1.00 | 1.00 |
| Perfume | 0.50 | 0.50 | 0.50 | 0.50 | 0.50 | 0.50 |
| Purified water | 3.00 | 3.00 | 3.00 | 3.00 | 3.00 | 3.00 |
| Copolymer 1 | 10.00 | | | | | |
| Copolymer 2 | | 10.00 | | | | |
| Copolymer 3 | | | 10.00 | | | |
| Copolymer 4 | | | | 10.00 | | |
| Copolymer 5 | | | | | 10.00 | |
| Copolymer 6 | | | | | | 10.00 |
| Cetyl ethylhexanoate | Balance | Balance | Balance | Balance | Balance | Balance |
| Total | 100 | 100 | 100 | 100 | 100 | 100 |

**Table 8**

| Mascara | | | | | | |
|---|---|---|---|---|---|---|
| | Formulation Example 37 | Formulation Example 38 | Formulation Example 39 | Formulation Example 40 | Formulation Example 41 | Formulation Example 42 |
| Cetyl PEG/PPG-10/1 dimethicone | 2.00 | 2.00 | 2.00 | 2.00 | 2.00 | 2.00 |
| Trimethylsiloxysilicic acid | 4.00 | 4.00 | 4.00 | 4.00 | 4.00 | 4.00 |
| Polyethylene | 5.00 | 5.00 | 5.00 | 5.00 | 5.00 | 5.00 |
| Dextrin palmitate | 0.50 | 0.50 | 0.50 | 0.50 | 0.50 | 0.50 |
| Lecithin | 0.50 | 0.50 | 0.50 | 0.50 | 0.50 | 0.50 |
| Sucrose acetate isobutyrate | 2.50 | 2.50 | 2.50 | 2.50 | 2.50 | 2.50 |
| Beeswax | 7.00 | 7.00 | 7.00 | 7.00 | 7.00 | 7.00 |
| Cyclopentasiloxane | 5.00 | 5.00 | 5.00 | 5.00 | 5.00 | 5.00 |
| Black iron oxide | 3.00 | 3.00 | 3.00 | 3.00 | 3.00 | 3.00 |
| Silica | 4.00 | 4.00 | 4.00 | 4.00 | 4.00 | 4.00 |
| Talc | 10.00 | 10.00 | 10.00 | 10.00 | 10.00 | 10.00 |
| Copolymer 1 | 40.00 | | | | | |
| Copolymer 2 | | 40.00 | | | | |
| Copolymer 3 | | | 35.00 | | | |
| Copolymer 4 | | | | 35.00 | | |
| Copolymer 5 | | | | | 30.00 | |
| Copolymer 6 | | | | | | 30.00 |
| BG | 2.00 | 2.00 | 2.00 | 2.00 | 2.00 | 2.00 |
| Phenoxyethanol | 0.50 | 0.50 | 0.50 | 0.50 | 0.50 | 0.50 |
| Purified water | Balance | Balance | Balance | Balance | Balance | Balance |
| Total | 100 | 100 | 100 | 100 | 100 | 100 |

**Table 9**

| Eyeliner | | | | | | |
|---|---|---|---|---|---|---|
| | Formulation Example 43 | Formulation Example 44 | Formulation Example 45 | Formulation Example 46 | Formulation Example 47 | Formulation Example 48 |
| Cetyl PEG/PPG-10/1 dimethicone | 2.00 | 2.00 | 2.00 | 2.00 | 2.00 | 2.00 |
| Trimethylsiloxysilicic acid | 7.00 | 7.00 | 7.00 | 7.00 | 7.00 | 7.00 |
| Polyethylene | 2.00 | 2.00 | 2.00 | 2.00 | 2.00 | 2.00 |
| Dextrin palmitate | 0.50 | 0.50 | 0.50 | 0.50 | 0.50 | 0.50 |
| Lecithin | 0.30 | 0.30 | 0.30 | 0.30 | 0.30 | 0.30 |
| Beeswax | 3.00 | 3.00 | 3.00 | 3.00 | 3.00 | 3.00 |
| Cyclopentasiloxane | 35.00 | 35.00 | 35.00 | 35.00 | 35.00 | 35.00 |
| Disteardimonium hectorite | 1.00 | 1.00 | 1.00 | 1.00 | 1.00 | 1.00 |
| Black iron oxide | 10.00 | 10.00 | 10.00 | 10.00 | 10.00 | 10.00 |
| Titanium oxide | 1.00 | 1.00 | 1.00 | 1.00 | 1.00 | 1.00 |
| Copolymer 1 | 15.00 | | | | | |
| Copolymer 2 | | 15.00 | | | | |
| Copolymer 3 | | | 12.50 | | | |
| Copolymer 4 | | | | 12.50 | | |
| Copolymer 5 | | | | | 10.00 | |
| Copolymer 6 | | | | | | 10.00 |
| BG | 2.00 | 2.00 | 2.00 | 2.00 | 2.00 | 2.00 |
| Phenoxyethanol | 0.50 | 0.50 | 0.50 | 0.50 | 0.50 | 0.50 |
| Purified water | Balance | Balance | Balance | Balance | Balance | Balance |
| Total | 100 | 100 | 100 | 100 | 100 | 100 |

**Table 10**

| Water-in-oil type liquid foundation | | | | | | |
|---|---|---|---|---|---|---|
| | Formulation Example 49 | Formulation Example 50 | Formulation Example 51 | Formulation Example 52 | Formulation Example 53 | Formulation Example 54 |
| Cetyl PEG/PPG-10/1 dimethicone | 2.00 | 2.00 | 2.00 | 2.00 | 2.00 | 2.00 |
| Microcrystalline wax | 1.00 | 1.00 | 1.00 | 1.00 | 1.00 | 1.00 |
| Ceresin | 5.00 | 5.00 | 5.00 | 5.00 | 5.00 | 5.00 |
| Paraffin | 4.00 | 4.00 | 4.00 | 4.00 | 4.00 | 4.00 |
| PG dicaprate | 2.00 | 2.00 | 2.00 | 2.00 | 2.00 | 2.00 |
| Dimethicone | 21.00 | 21.00 | 21.00 | 21.00 | 21.00 | 21.00 |
| Sorbitan sesquioleate | 0.20 | 0.20 | 0.20 | 0.20 | 0.20 | 0.20 |
| Lecithin | 0.20 | 0.20 | 0.20 | 0.20 | 0.20 | 0.20 |
| Titanium oxide | 10.00 | 10.00 | 10.00 | 10.00 | 10.00 | 10.00 |
| Colcothar | 0.40 | 0.40 | 0.40 | 0.40 | 0.40 | 0.40 |
| Yellow iron oxide | 2.00 | 2.00 | 2.00 | 2.00 | 2.00 | 2.00 |
| Black iron oxide | 0.10 | 0.10 | 0.10 | 0.10 | 0.10 | 0.10 |
| Copolymer 1 | 3.00 | | | | | |
| Copolymer 2 | | 3.00 | | | | |
| Copolymer 3 | | | 3.00 | | | |
| Copolymer 4 | | | | 3.00 | | |
| Copolymer 5 | | | | | 3.00 | |
| Copolymer 6 | | | | | | 3.00 |
| BG | 1.00 | 1.00 | 1.00 | 1.00 | 1.00 | 1.00 |
| Phenoxyethanol | 0.50 | 0.50 | 0.50 | 0.50 | 0.50 | 0.50 |
| Purified water | Balance | Balance | Balance | Balance | Balance | Balance |
| Total | 100 | 100 | 100 | 100 | 100 | 100 |

**Table 11**

| Oil-in-water type liquid foundation | | | | | | |
|---|---|---|---|---|---|---|
| | Formulation Example 55 | Formulation Example 56 | Formulation Example 57 | Formulation Example 58 | Formulation Example 59 | Formulation Example 60 |
| Polysorbate 60 | 0.50 | 0.50 | 0.50 | 0.50 | 0.50 | 0.50 |
| Sorbitan sesquioleate | 1.00 | 1.00 | 1.00 | 1.00 | 1.00 | 1.00 |
| Ethylhexyl methoxycinnamate | 5.00 | 5.00 | 5.00 | 5.00 | 5.00 | 5.00 |
| Cyclopentasiloxane | 5.00 | 5.00 | 5.00 | 5.00 | 5.00 | 5.00 |
| Triethylhexanoin | 3.00 | 3.00 | 3.00 | 3.00 | 3.00 | 3.00 |
| Hydrogenated polyisobutene | 10.00 | 10.00 | 10.00 | 10.00 | 10.00 | 10.00 |
| Dimethicone | 1.00 | 1.00 | 1.00 | 1.00 | 1.00 | 1.00 |
| Isononyl isononanoate | 2.00 | 2.00 | 2.00 | 2.00 | 2.00 | 2.00 |
| Diisostearyl malate | 0.25 | 0.25 | 0.25 | 0.25 | 0.25 | 0.25 |
| Titanium oxide | 5.00 | 5.00 | 5.00 | 5.00 | 5.00 | 5.00 |
| Colcothar | 0.10 | 0.10 | 0.10 | 0.10 | 0.10 | 0.10 |
| Yellow iron oxide | 0.25 | 0.25 | 0.25 | 0.25 | 0.25 | 0.25 |
| Black iron oxide | 0.01 | 0.01 | 0.01 | 0.01 | 0.01 | 0.01 |
| Copolymer 1 | 2.00 | | | | | |
| Copolymer 2 | | 2.00 | | | | |
| Copolymer 3 | | | 2.00 | | | |
| Copolymer 4 | | | | 2.00 | | |
| Copolymer 5 | | | | | 2.00 | |
| Copolymer 6 | | | | | | 2.00 |
| BG | 7.50 | 7.50 | 7.50 | 7.50 | 7.50 | 7.50 |
| Glycerin | 4.00 | 4.00 | 4.00 | 4.00 | 4.00 | 4.00 |
| (PEG-240/decyltetradeceth-20/HDI) copolymer | 0.90 | 0.90 | 0.90 | 0.90 | 0.90 | 0.90 |
| Xanthan gum | 0.05 | 0.05 | 0.05 | 0.05 | 0.05 | 0.05 |
| Hydroxypropylmethylcellulose | 0.05 | 0.05 | 0.05 | 0.05 | 0.05 | 0.05 |
| Purified water | Balance | Balance | Balance | Balance | Balance | Balance |
| Total | 100 | 100 | 100 | 100 | 100 | 100 |

**Table 12**

| Powder foundation | | | | | | |
|---|---|---|---|---|---|---|
| | Formulation Example 61 | Formulation Example 62 | Formulation Example 63 | Formulation Example 64 | Formulation Example 65 | Formulation Example 66 |
| (Dimethicone/phenyl vinyl dimethicone) crosspolymer | 5.00 | 5.00 | 5.00 | 5.00 | 5.00 | 5.00 |
| Diisostearyl malate | 5.00 | 5.00 | 5.00 | 5.00 | 5.00 | 5.00 |
| Titanium oxide treated with 2% triethoxycaprylylsilane | 15.00 | 15.00 | 15.00 | 15.00 | 15.00 | 15.00 |
| Colcothar | 1.00 | 1.00 | 1.00 | 1.00 | 1.00 | 1.00 |
| Yellow iron oxide | 3.00 | 3.00 | 3.00 | 3.00 | 3.00 | 3.00 |
| Black iron oxide | 0.50 | 0.50 | 0.50 | 0.50 | 0.50 | 0.50 |
| Methyl polymethyl methacrylate | 5.00 | 5.00 | 5.00 | 5.00 | 5.00 | 5.00 |
| Talc | 10.00 | 10.00 | 10.00 | 10.00 | 10.00 | 10.00 |
| Silica | 5.00 | 5.00 | 5.00 | 5.00 | 5.00 | 5.00 |
| Lauroyl lysine | 5.00 | 5.00 | 5.00 | 5.00 | 5.00 | 5.00 |
| Ba sulfate | 5.00 | 5.00 | 5.00 | 5.00 | 5.00 | 5.00 |
| Zinc stearate | 5.00 | 5.00 | 5.00 | 5.00 | 5.00 | 5.00 |
| Copolymer 1 | 1.00 | | | | | |
| Copolymer 2 | | 1.00 | | | | |
| Copolymer 3 | | | 1.00 | | | |
| Copolymer 4 | | | | 1.00 | | |
| Copolymer 5 | | | | | 1.00 | |
| Copolymer 6 | | | | | | 1.00 |
| Mica | Balance | Balance | Balance | Balance | Balance | Balance |
| Total | 100 | 100 | 100 | 100 | 100 | 100 |

**Table 13**

| Hair oil | | | | | | |
|---|---|---|---|---|---|---|
| | Formulation Example 67 | Formulation Example 68 | Formulation Example 69 | Formulation Example 70 | Formulation Example 71 | Formulation Example 72 |
| Isododecane | 40.00 | 40.00 | 40.00 | 40.00 | 40.00 | 40.00 |
| Dimethiconol | 10.00 | 10.00 | 10.00 | 10.00 | 10.00 | 10.00 |
| Diphenylsiloxy phenyl trimethicone | 5.00 | 5.00 | 5.00 | 5.00 | 5.00 | 5.00 |
| Perfume | 0.10 | 0.10 | 0.10 | 0.10 | 0.10 | 0.10 |
| Copolymer 1 | 5.00 | | | | | |
| Copolymer 2 | | 5.00 | | | | |
| Copolymer 3 | | | 5.00 | | | |
| Copolymer 4 | | | | 5.00 | | |
| Copolymer 5 | | | | | 5.00 | |
| Copolymer 6 | | | | | | 5.00 |
| Hydrogenated polyisobutene | Balance | Balance | Balance | Balance | Balance | Balance |
| Total | 100 | 100 | 100 | 100 | 100 | 100 |

**Table 14**

| Hair milk | | | | | | |
|---|---|---|---|---|---|---|
| | Formulation Example 73 | Formulation Example 74 | Formulation Example 75 | Formulation Example 76 | Formulation Example 77 | Formulation Example 78 |
| Stearyl alcohol | 3.00 | 3.00 | 3.00 | 3.00 | 3.00 | 3.00 |
| Ethylhexyl palmitate | 2.00 | 2.00 | 2.00 | 2.00 | 2.00 | 2.00 |
| Triethylhexanoin | 2.00 | 2.00 | 2.00 | 2.00 | 2.00 | 2.00 |
| Glyceryl stearate | 1.50 | 1.50 | 1.50 | 1.50 | 1.50 | 1.50 |
| PEG-40 stearate | 1.50 | 1.50 | 1.50 | 1.50 | 1.50 | 1.50 |
| Amodimethicone | 0.80 | 0.80 | 0.80 | 0.80 | 0.80 | 0.80 |
| Perfume | 0.30 | 0.30 | 0.30 | 0.30 | 0.30 | 0.30 |
| Copolymer 1 | 5.00 | | | | | |
| Copolymer 2 | | 5.00 | | | | |
| Copolymer 3 | | | 5.00 | | | |
| Copolymer 4 | | | | 5.00 | | |
| Copolymer 5 | | | | | 5.00 | |
| Copolymer 6 | | | | | | 5.00 |
| Phenoxyethanol | 0.50 | 0.50 | 0.50 | 0.50 | 0.50 | 0.50 |
| Glycerin | 3.00 | 3.00 | 3.00 | 3.00 | 3.00 | 3.00 |
| Hydroxyethylcellulose | 0.20 | 0.20 | 0.20 | 0.20 | 0.20 | 0.20 |
| Steartrimonium chloride | 0.56 | 0.56 | 0.56 | 0.56 | 0.56 | 0.56 |
| Purified water | Balance | Balance | Balance | Balance | Balance | Balance |
| Total | 100 | 100 | 100 | 100 | 100 | 100 |

**Table 15**

| Hair shampoo | | | | | | |
|---|---|---|---|---|---|---|
| | Formulation Example 79 | Formulation Example 80 | Formulation Example 81 | Formulation Example 82 | Formulation Example 83 | Formulation Example 84 |
| TEA cocoyl glutamate | 5.00 | 5.00 | 5.00 | 5.00 | 5.00 | 5.00 |
| Cocamidopropylbetaine | 4.00 | 4.00 | 4.00 | 4.00 | 4.00 | 4.00 |
| Polyquaternium-10 | 0.50 | 0.50 | 0.50 | 0.50 | 0.50 | 0.50 |
| PPG-2 cocamide | 3.00 | 3.00 | 3.00 | 3.00 | 3.00 | 3.00 |
| Citric acid | 0.10 | 0.10 | 0.10 | 0.10 | 0.10 | 0.10 |
| Methylparaben | 0.20 | 0.20 | 0.20 | 0.20 | 0.20 | 0.20 |
| Perfume | 0.50 | 0.50 | 0.50 | 0.50 | 0.50 | 0.50 |
| Copolymer 1 | 1.00 | | | | | |
| Copolymer 2 | | 1.00 | | | | |
| Copolymer 3 | | | 1.00 | | | |
| Copolymer 4 | | | | 1.00 | | |
| Copolymer 5 | | | | | 1.00 | |
| Copolymer 6 | | | | | | 1.00 |
| Purified water | Balance | Balance | Balance | Balance | Balance | Balance |
| Total | 100 | 100 | 100 | 100 | 100 | 100 |

**Table 16**

| Hair conditioner | | | | | | |
|---|---|---|---|---|---|---|
| | Formulation Example 85 | Formulation Example 86 | Formulation Example 87 | Formulation Example 88 | Formulation Example 89 | Formulation Example 90 |
| Stearamidopropyl dimethylamine | 1.20 | 1.20 | 1.20 | 1.20 | 1.20 | 1.20 |
| Stearyl alcohol | 4.50 | 4.50 | 4.50 | 4.50 | 4.50 | 4.50 |
| Dimethicone | 3.00 | 3.00 | 3.00 | 3.00 | 3.00 | 3.00 |
| Perfume | 0.30 | 0.30 | 0.30 | 0.30 | 0.30 | 0.30 |
| Copolymer 1 | 3.00 | | | | | |
| Copolymer 2 | | 3.00 | | | | |
| Copolymer 3 | | | 3.00 | | | |
| Copolymer 4 | | | | 3.00 | | |
| Copolymer 5 | | | | | 3.00 | |
| Copolymer 6 | | | | | | 3.00 |
| DPG | 2.00 | 2.00 | 2.00 | 2.00 | 2.00 | 2.00 |
| Hydroxyethylcellulose | 0.50 | 0.50 | 0.50 | 0.50 | 0.50 | 0.50 |
| Lactic acid | 0.70 | 0.70 | 0.70 | 0.70 | 0.70 | 0.70 |
| Phenoxyethanol | 0.50 | 0.50 | 0.50 | 0.50 | 0.50 | 0.50 |
| Purified water | Balance | Balance | Balance | Balance | Balance | Balance |
| Total | 100 | 100 | 100 | 100 | 100 | 100 |

**Table 17**

| Hair color treatment | | | | | | |
|---|---|---|---|---|---|---|
| | Formulation Example 91 | Formulation Example 92 | Formulation Example 93 | Formulation Example 94 | Formulation Example 95 | Formulation Example 96 |
| Glycerin | 1.00 | 1.00 | 1.00 | 1.00 | 1.00 | 1.00 |
| 1,3-Butylene glycol | 1.00 | 1.00 | 1.00 | 1.00 | 1.00 | 1.00 |
| Pentylene glycol | 1.00 | 1.00 | 1.00 | 1.00 | 1.00 | 1.00 |
| Hydroxyethylcellulose | 0.25 | 0.25 | 0.25 | 0.25 | 0.25 | 0.25 |
| Steartrimonium bromide | 1.50 | 1.50 | 1.50 | 1.50 | 1.50 | 1.50 |
| Myristyl alcohol | 5.00 | 5.00 | 5.00 | 5.00 | 5.00 | 5.00 |
| Behenyl alcohol | 1.00 | 1.00 | 1.00 | 1.00 | 1.00 | 1.00 |
| Ethylhexyl palmitate | 2.00 | 2.00 | 2.00 | 2.00 | 2.00 | 2.00 |
| Cetyl palmitate | 1.00 | 1.00 | 1.00 | 1.00 | 1.00 | 1.00 |
| Glycol stearate | 2.00 | 2.00 | 2.00 | 2.00 | 2.00 | 2.00 |
| Shea butter | 1.00 | 1.00 | 1.00 | 1.00 | 1.00 | 1.00 |
| Ammonium hydrogen carbonate | 0.70 | 0.70 | 0.70 | 0.70 | 0.70 | 0.70 |
| Menthol | 0.10 | 0.10 | 0.10 | 0.10 | 0.10 | 0.10 |
| Ethanol | 1.00 | 1.00 | 1.00 | 1.00 | 1.00 | 1.00 |
| Dipotassium glycyrrhizate | 0.10 | 0.10 | 0.10 | 0.10 | 0.10 | 0.10 |
| Basic Blue No. 99 | 0.30 | 0.30 | 0.30 | 0.30 | 0.30 | 0.30 |
| Basic Brown No. 16 | 0.50 | 0.50 | 0.50 | 0.50 | 0.50 | 0.50 |
| HC Blue No. 2 | 0.50 | 0.50 | 0.50 | 0.50 | 0.50 | 0.50 |
| HC Yellow No. 4 | 0.20 | 0.20 | 0.20 | 0.20 | 0.20 | 0.20 |
| HC Yellow No. 2 | 0.10 | 0.10 | 0.10 | 0.10 | 0.10 | 0.10 |
| Perfume | 0.30 | 0.30 | 0.30 | 0.30 | 0.30 | 0.30 |
| Copolymer 1 | 2.00 | | | | | |
| Copolymer 2 | | 2.00 | | | | |
| Copolymer 3 | | | 2.00 | | | |
| Copolymer 4 | | | | 2.00 | | |
| Copolymer 5 | | | | | 2.00 | |
| Copolymer 6 | | | | | | 2.00 |
| Purified water | Balance | Balance | Balance | Balance | Balance | Balance |
| Total | 100 | 100 | 100 | 100 | 100 | 100 |

**Table 18**

| Hair manicure | | | | | | |
|---|---|---|---|---|---|---|
| | Formulation Example 97 | Formulation Example 98 | Formulation Example 99 | Formulation Example 100 | Formulation Example 101 | Formulation Example 102 |
| Cetyl alcohol | 8.00 | 8.00 | 8.00 | 8.00 | 8.00 | 8.00 |
| Stearic acid | 2.00 | 2.00 | 2.00 | 2.00 | 2.00 | 2.00 |
| Ceteth-20 | 5.00 | 5.00 | 5.00 | 5.00 | 5.00 | 5.00 |
| Sodium lauryl sulfate | 0.50 | 0.50 | 0.50 | 0.50 | 0.50 | 0.50 |
| Copolymer 1 | 0.50 | | | | | |
| Copolymer 2 | | 0.50 | | | | |
| Copolymer 3 | | | 0.50 | | | |
| Copolymer 4 | | | | 0.50 | | |
| Copolymer 5 | | | | | 0.50 | |
| Copolymer 6 | | | | | | 0.50 |
| Black No. 401 | 0.02 | 0.02 | 0.02 | 0.02 | 0.02 | 0.02 |
| Purple No. 401 | 0.02 | 0.02 | 0.02 | 0.02 | 0.02 | 0.02 |
| Orange No. 205 | 0.08 | 0.08 | 0.08 | 0.08 | 0.08 | 0.08 |
| Perfume | 0.30 | 0.30 | 0.30 | 0.30 | 0.30 | 0.30 |
| PG | 3.00 | 3.00 | 3.00 | 3.00 | 3.00 | 3.00 |
| Xanthan gum | 1.00 | 1.00 | 1.00 | 1.00 | 1.00 | 1.00 |
| Citric acid | 2.00 | 2.00 | 2.00 | 2.00 | 2.00 | 2.00 |
| Benzyl alcohol | 4.00 | 4.00 | 4.00 | 4.00 | 4.00 | 4.00 |
| Purified water | Balance | Balance | Balance | Balance | Balance | Balance |
| Total | 100 | 100 | 100 | 100 | 100 | 100 |

**Table 19**

| Hair wax | | | | | | |
|---|---|---|---|---|---|---|
| | Formulation Example 103 | Formulation Example 104 | Formulation Example 105 | Formulation Example 106 | Formulation Example 107 | Formulation Example 108 |
| Carnauba wax | 6.00 | 6.00 | 6.00 | 6.00 | 6.00 | 6.00 |
| Candelilla wax | 2.00 | 2.00 | 2.00 | 2.00 | 2.00 | 2.00 |
| Glyceryl isostearate | 1.00 | 1.00 | 1.00 | 1.00 | 1.00 | 1.00 |
| PEG-8 | 1.00 | 1.00 | 1.00 | 1.00 | 1.00 | 1.00 |
| Isostearic acid | 2.00 | 2.00 | 2.00 | 2.00 | 2.00 | 2.00 |
| Cyclopentanesiloxane | 5.00 | 5.00 | 5.00 | 5.00 | 5.00 | 5.00 |
| Polyethylene | 2.00 | 2.00 | 2.00 | 2.00 | 2.00 | 2.00 |
| Mineral oil | 10.00 | 10.00 | 10.00 | 10.00 | 10.00 | 10.00 |
| Perfume | 0.20 | 0.20 | 0.20 | 0.20 | 0.20 | 0.20 |
| Copolymer 1 | 2.00 | | | | | |
| Copolymer 2 | | 2.00 | | | | |
| Copolymer 3 | | | 2.00 | | | |
| Copolymer 4 | | | | 2.00 | | |
| Copolymer 5 | | | | | 2.00 | |
| Copolymer 6 | | | | | | 2.00 |
| Carbomer | 0.10 | 0.10 | 0.10 | 0.10 | 0.10 | 0.10 |
| TEA | 0.10 | 0.10 | 0.10 | 0.10 | 0.10 | 0.10 |
| Phenoxyethanol | 0.50 | 0.50 | 0.50 | 0.50 | 0.50 | 0.50 |
| Purified water | Balance | Balance | Balance | Balance | Balance | Balance |
| Total | 100 | 100 | 100 | 100 | 100 | 100 |

**Table 20**

| Cleansing oil | | | | | | |
|---|---|---|---|---|---|---|
| | Formulation Example 109 | Formulation Example 110 | Formulation Example 111 | Formulation Example 112 | Formulation Example 113 | Formulation Example 114 |
| PEG-20 glyceryl triisostearate | 10.00 | 10.00 | 10.00 | 10.00 | 10.00 | 10.00 |
| PEG-5 glyceryl triisostearate | 2.00 | 2.00 | 2.00 | 2.00 | 2.00 | 2.00 |
| Glyceryl tri(caprylate/caprate) | 10.00 | 10.00 | 10.00 | 10.00 | 10.00 | 10.00 |
| Copolymer 1 | 10.00 | | | | | |
| Copolymer 2 | | 10.00 | | | | |
| Copolymer 3 | | | 10.00 | | | |
| Copolymer 4 | | | | 10.00 | | |
| Copolymer 5 | | | | | 10.00 | |
| Copolymer 6 | | | | | | 10.00 |
| Cetyl ethylhexanoate | Balance | Balance | Balance | Balance | Balance | Balance |
| Total | 100 | 100 | 100 | 100 | 100 | 100 |

**Table 21**

| Cleansing balm | | | | | | |
|---|---|---|---|---|---|---|
| | Formulation Example 115 | Formulation Example 116 | Formulation Example 117 | Formulation Example 118 | Formulation Example 119 | Formulation Example 120 |
| PEG-20 glyceryl triisostearate | 12.00 | 12.00 | 12.00 | 12.00 | 12.00 | 12.00 |
| PEG-5 glyceryl triisostearate | 3.00 | 3.00 | 3.00 | 3.00 | 3.00 | 3.00 |
| Ethylhexyl palmitate | 25.00 | 25.00 | 25.00 | 25.00 | 25.00 | 25.00 |
| Microcrystalline wax | 10.00 | 10.00 | 10.00 | 10.00 | 10.00 | 10.00 |
| Copolymer 1 | 5.00 | | | | | |
| Copolymer 2 | | 5.00 | | | | |
| Copolymer 3 | | | 5.00 | | | |
| Copolymer 4 | | | | 5.00 | | |
| Copolymer 5 | | | | | 5.00 | |
| Copolymer 6 | | | | | | 5.00 |
| Glyceryl tri(caprylate/caprate) | Balance | Balance | Balance | Balance | Balance | Balance |
| Total | 100 | 100 | 100 | 100 | 100 | 100 |

**Table 22**

| Oil-in-water type cream | | | | | | |
|---|---|---|---|---|---|---|
| | Formulation Example 121 | Formulation Example 122 | Formulation Example 123 | Formulation Example 124 | Formulation Example 125 | Formulation Example 126 |
| Glyceryl tri(caprylate/caprate) | 10.00 | 10.00 | 10.00 | 10.00 | 10.00 | 10.00 |
| Mineral oil | 10.00 | 10.00 | 10.00 | 10.00 | 10.00 | 10.00 |
| Glyceryl stearate (SE) | 4.00 | 4.00 | 4.00 | 4.00 | 4.00 | 4.00 |
| Cetearyl alcohol | 2.00 | 2.00 | 2.00 | 2.00 | 2.00 | 2.00 |
| Copolymer 1 | 2.00 | | | | | |
| Copolymer 2 | | 2.00 | | | | |
| Copolymer 3 | | | 2.00 | | | |
| Copolymer 4 | | | | 2.00 | | |
| Copolymer 5 | | | | | 2.00 | |
| Copolymer 6 | | | | | | 2.00 |
| Glycerin | 2.00 | 2.00 | 2.00 | 2.00 | 2.00 | 2.00 |
| Xanthan gum | 0.10 | 0.10 | 0.10 | 0.10 | 0.10 | 0.10 |
| Carbomer | 0.10 | 0.10 | 0.10 | 0.10 | 0.10 | 0.10 |
| Phenoxyethanol | 0.50 | 0.50 | 0.50 | 0.50 | 0.50 | 0.50 |
| Sodium hydroxide | 0.05 | 0.05 | 0.05 | 0.05 | 0.05 | 0.05 |
| Purified water | Balance | Balance | Balance | Balance | Balance | Balance |
| Total | 100 | 100 | 100 | 100 | 100 | 100 |

**Table 23**

| Water-in-oil type cream | | | | | | |
|---|---|---|---|---|---|---|
| | Formulation Example 127 | Formulation Example 128 | Formulation Example 129 | Formulation Example 130 | Formulation Example 131 | Formulation Example 132 |
| Lauryl PEG-9 polydimethylsiloxyethyl dimethicone | 2.00 | 2.00 | 2.00 | 2.00 | 2.00 | 2.00 |
| Disteardimonium hectorite | 1.50 | 1.50 | 1.50 | 1.50 | 1.50 | 1.50 |
| Triethylhexanoin | 10.00 | 10.00 | 10.00 | 10.00 | 10.00 | 10.00 |
| Dimethicone | 15.00 | 15.00 | 15.00 | 15.00 | 15.00 | 15.00 |
| Cyclopentasiloxane | 5.00 | 5.00 | 5.00 | 5.00 | 5.00 | 5.00 |
| Copolymer 1 | 5.00 | | | | | |
| Copolymer 2 | | 5.00 | | | | |
| Copolymer 3 | | | 5.00 | | | |
| Copolymer 4 | | | | 5.00 | | |
| Copolymer 5 | | | | | 5.00 | |
| Copolymer 6 | | | | | | 5.00 |
| BG | 1.00 | 1.00 | 1.00 | 1.00 | 1.00 | 1.00 |
| Phenoxyethanol | 0.50 | 0.50 | 0.50 | 0.50 | 0.50 | 0.50 |
| Purified water | Balance | Balance | Balance | Balance | Balance | Balance |
| Total | 100 | 100 | 100 | 100 | 100 | 100 |

**Table 24**

| Sunscreen gel | | | | | | |
|---|---|---|---|---|---|---|
| | Formulation Example 133 | Formulation Example 134 | Formulation Example 135 | Formulation Example 136 | Formulation Example 137 | Formulation Example 138 |
| Alkyl (C12-15) benzoate | 3.00 | 3.00 | 3.00 | 3.00 | 3.00 | 3.00 |
| Ethylhexyl methoxycinnamate | 7.00 | 7.00 | 7.00 | 7.00 | 7.00 | 7.00 |
| Ethylhexyl triazone | 2.00 | 2.00 | 2.00 | 2.00 | 2.00 | 2.00 |
| t-Butyl methoxydibenzoylmethane | 1.00 | 1.00 | 1.00 | 1.00 | 1.00 | 1.00 |
| PEG-60 hydrogenated castor oil | 1.00 | 1.00 | 1.00 | 1.00 | 1.00 | 1.00 |
| Copolymer 1 | 3.00 | | | | | |
| Copolymer 2 | | 3.00 | | | | |
| Copolymer 3 | | | 3.00 | | | |
| Copolymer 4 | | | | 3.00 | | |
| Copolymer 5 | | | | | 3.00 | |
| Copolymer 6 | | | | | | 3.00 |
| BG | 1.50 | 1.50 | 1.50 | 1.50 | 1.50 | 1.50 |
| (Acrylate/alkyl acrylate (C10-30)) crosspolymer | 0.30 | 0.30 | 0.30 | 0.30 | 0.30 | 0.30 |
| Ethanol | 5.00 | 5.00 | 5.00 | 5.00 | 5.00 | 5.00 |
| Phenoxyethanol | 0.50 | 0.50 | 0.50 | 0.50 | 0.50 | 0.50 |
| Potassium hydroxide | 0.08 | 0.08 | 0.08 | 0.08 | 0.08 | 0.08 |
| Purified water | Balance | Balance | Balance | Balance | Balance | Balance |
| Total | 100 | 100 | 100 | 100 | 100 | 100 |

**Table 25**

| Oil-in-water type sunscreen cream | | | | | | |
|---|---|---|---|---|---|---|
| | Formulation Example 139 | Formulation Example 140 | Formulation Example 141 | Formulation Example 142 | Formulation Example 143 | Formulation Example 144 |
| Behenyl alcohol | 0.50 | 0.50 | 0.50 | 0.50 | 0.50 | 0.50 |
| Cetostearyl alcohol | 2.00 | 2.00 | 2.00 | 2.00 | 2.00 | 2.00 |
| Glyceryl stearate | 1.50 | 1.50 | 1.50 | 1.50 | 1.50 | 1.50 |
| Cetyl ethylhexanoate | 3.50 | 3.50 | 3.50 | 3.50 | 3.50 | 3.50 |
| Isostearic acid | 3.00 | 3.00 | 3.00 | 3.00 | 3.00 | 3.00 |
| Dimethicone | 2.00 | 2.00 | 2.00 | 2.00 | 2.00 | 2.00 |
| Cyclopentasiloxane | 20.50 | 20.50 | 20.50 | 20.50 | 20.50 | 20.50 |
| Polysorbate 80 | 2.00 | 2.00 | 2.00 | 2.00 | 2.00 | 2.00 |
| Hydrophobic-treated titanium oxide | 15.00 | 15.00 | 15.00 | 15.00 | 15.00 | 15.00 |
| Potassium cetyl phosphate | 1.00 | 1.00 | 1.00 | 1.00 | 1.00 | 1.00 |
| Copolymer 1 | 2.00 | | | | | |
| Copolymer 2 | | 2.00 | | | | |
| Copolymer 3 | | | 2.00 | | | |
| Copolymer 4 | | | | 2.00 | | |
| Copolymer 5 | | | | | 2.00 | |
| Copolymer 6 | | | | | | 2.00 |
| BG | 10.00 | 10.00 | 10.00 | 10.00 | 10.00 | 10.00 |
| Xanthan gum | 0.20 | 0.20 | 0.20 | 0.20 | 0.20 | 0.20 |
| Phenoxyethanol | 0.70 | 0.70 | 0.70 | 0.70 | 0.70 | 0.70 |
| Citric acid | 0.01 | 0.01 | 0.01 | 0.01 | 0.01 | 0.01 |
| Purified water | Balance | Balance | Balance | Balance | Balance | Balance |
| Total | 100 | 100 | 100 | 100 | 100 | 100 |

**Table 26**

| Water-in-oil type sunscreen cream | | | | | | |
|---|---|---|---|---|---|---|
| | Formulation Example 145 | Formulation Example 146 | Formulation Example 147 | Formulation Example 148 | Formulation Example 149 | Formulation Example 150 |
| Lauryl PEG-9 polydimethylsiloxyethyl dimethicone | 2.00 | 2.00 | 2.00 | 2.00 | 2.00 | 2.00 |
| Stearalkonium hectorite | 0.50 | 0.50 | 0.50 | 0.50 | 0.50 | 0.50 |
| Dextrin palmitate | 0.50 | 0.50 | 0.50 | 0.50 | 0.50 | 0.50 |
| Cetyl ethylhexanoate | 20.00 | 20.00 | 20.00 | 20.00 | 20.00 | 20.00 |
| Triethylhexanoin | 3.00 | 3.00 | 3.00 | 3.00 | 3.00 | 3.00 |
| Cyclopentasiloxane | 20.00 | 20.00 | 20.00 | 20.00 | 20.00 | 20.00 |
| Ethylhexyl methoxycinnamate | 5.00 | 5.00 | 5.00 | 5.00 | 5.00 | 5.00 |
| Zinc oxide | 20.00 | 20.00 | 20.00 | 20.00 | 20.00 | 20.00 |
| Titanium oxide | 10.00 | 10.00 | 10.00 | 10.00 | 10.00 | 10.00 |
| Trimethylsiloxysilicic acid | 4.00 | 4.00 | 4.00 | 4.00 | 4.00 | 4.00 |
| Copolymer 1 | 4.00 | | | | | |
| Copolymer 2 | | 4.00 | | | | |
| Copolymer 3 | | | 4.00 | | | |
| Copolymer 4 | | | | 4.00 | | |
| Copolymer 5 | | | | | 4.00 | |
| Copolymer 6 | | | | | | 4.00 |
| BG | 1.00 | 1.00 | 1.00 | 1.00 | 1.00 | 1.00 |
| Phenoxyethanol | 0.50 | 0.50 | 0.50 | 0.50 | 0.50 | 0.50 |
| Purified water | Balance | Balance | Balance | Balance | Balance | Balance |
| Total | 100 | 100 | 100 | 100 | 100 | 100 |

**Table 27**

| Body oil | | | | | | |
|---|---|---|---|---|---|---|
| | Formulation Example 151 | Formulation Example 152 | Formulation Example 153 | Formulation Example 154 | Formulation Example 155 | Formulation Example 156 |
| Polyglyceryl-2 triisostearate | 3.00 | 3.00 | 3.00 | 3.00 | 3.00 | 3.00 |
| Di(phytosteryl/octyldodecyl) lauroyl glutamate | 5.00 | 5.00 | 5.00 | 5.00 | 5.00 | 5.00 |
| Tocopherol | 0.10 | 0.10 | 0.10 | 0.10 | 0.10 | 0.10 |
| DPG | 1.00 | 1.00 | 1.00 | 1.00 | 1.00 | 1.00 |
| Silica dimethyl silylate | 2.00 | 2.00 | 2.00 | 2.00 | 2.00 | 2.00 |
| Perfume | 0.05 | 0.05 | 0.05 | 0.05 | 0.05 | 0.05 |
| Copolymer 1 | 10.00 | | | | | |
| Copolymer 2 | | 10.00 | | | | |
| Copolymer 3 | | | 10.00 | | | |
| Copolymer 4 | | | | 10.00 | | |
| Copolymer 5 | | | | | 10.00 | |
| Copolymer 6 | | | | | | 10.00 |
| Olive oil | Balance | Balance | Balance | Balance | Balance | Balance |
| Total | 100 | 100 | 100 | 100 | 100 | 100 |

**Table 28**

| Body cream | | | | | | |
|---|---|---|---|---|---|---|
| | Formulation Example 157 | Formulation Example 158 | Formulation Example 159 | Formulation Example 160 | Formulation Example 161 | Formulation Example 162 |
| Polysorbate 60 | 1.00 | 1.00 | 1.00 | 1.00 | 1.00 | 1.00 |
| Sorbitan stearate | 1.00 | 1.00 | 1.00 | 1.00 | 1.00 | 1.00 |
| Isononyl isononanoate | 5.00 | 5.00 | 5.00 | 5.00 | 5.00 | 5.00 |
| Cetostearyl alcohol | 0.50 | 0.50 | 0.50 | 0.50 | 0.50 | 0.50 |
| Perfume | 0.20 | 0.20 | 0.20 | 0.20 | 0.20 | 0.20 |
| Copolymer 1 | 5.00 | | | | | |
| Copolymer 2 | | 5.00 | | | | |
| Copolymer 3 | | | 4.00 | | | |
| Copolymer 4 | | | | 4.00 | | |
| Copolymer 5 | | | | | 3.00 | |
| Copolymer 6 | | | | | | 3.00 |
| Glycerin | 3.00 | 3.00 | 3.00 | 3.00 | 3.00 | 3.00 |
| Ethanol | 3.00 | 3.00 | 3.00 | 3.00 | 3.00 | 3.00 |
| Phenoxyethanol | 0.50 | 0.50 | 0.50 | 0.50 | 0.50 | 0.50 |
| Carbomer | 0.20 | 0.20 | 0.20 | 0.20 | 0.20 | 0.20 |
| Sodium hydroxide | 0.10 | 0.10 | 0.10 | 0.10 | 0.10 | 0.10 |
| Purified water | Balance | Balance | Balance | Balance | Balance | Balance |
| Total | 100 | 100 | 100 | 100 | 100 | 100 |

**Table 29**

| Lip gloss 4 | | | | | | |
|---|---|---|---|---|---|---|
| | Formulation Example 163 | Formulation Example 164 | Formulation Example 165 | Formulation Example 166 | Formulation Example 167 | Formulation Example 168 |
| Ethylhexyl palmitate | 10.00 | 10.00 | 10.00 | 10.00 | 10.00 | 10.00 |
| Triethylhexanoin | 6.80 | 6.80 | 6.80 | 6.80 | 6.80 | 6.80 |
| Propylparaben | 0.20 | 0.20 | 0.20 | 0.20 | 0.20 | 0.20 |
| Dextrin palmitate | 3.00 | 3.00 | 3.00 | 3.00 | 3.00 | 3.00 |
| Red No. 201 | 0.80 | 0.80 | 0.80 | 0.80 | 0.80 | 0.80 |
| Red No. 202 | 0.80 | 0.80 | 0.80 | 0.80 | 0.80 | 0.80 |
| Polyglyceryl-2 triisostearate | 0.75 | 0.75 | 0.75 | 0.75 | 0.75 | 0.75 |
| Colcothar | 0.75 | 0.75 | 0.75 | 0.75 | 0.75 | 0.75 |
| Hydrophobic surface-treated titanium oxide | 2.20 | 2.20 | 2.20 | 2.20 | 2.20 | 2.20 |
| Copolymer 1 | 50.00 | | | | | |
| Copolymer 2 | | 50.00 | | | | |
| Copolymer 3 | | | 50.00 | | | |
| Copolymer 4 | | | | 40.00 | | |
| Copolymer 5 | | | | | 30.00 | |
| Copolymer 6 | | | | | | 20.00 |
| Diisostearyl malate | Balance | Balance | Balance | Balance | Balance | Balance |
| Total | 100 | 100 | 100 | 100 | 100 | 100 |

**Table 30**

| Lipstick 3 | | | | | | |
|---|---|---|---|---|---|---|
| | Formulation Example 169 | Formulation Example 170 | Formulation Example 171 | Formulation Example 172 | Formulation Example 173 | Formulation Example 174 |
| Synthesized wax | 8.00 | 8.00 | 8.00 | 8.00 | 8.00 | 8.00 |
| Polyethylene | 3.00 | 3.00 | 3.00 | 3.00 | 3.00 | 3.00 |
| Microcrystalline wax | 1.00 | 1.00 | 1.00 | 1.00 | 1.00 | 1.00 |
| Diisostearyl malate | 15.00 | 15.00 | 15.00 | 15.00 | 15.00 | 15.00 |
| Triethylhexanoin | 8.00 | 8.00 | 8.00 | 8.00 | 8.00 | 8.00 |
| Propylparaben | 0.20 | 0.20 | 0.20 | 0.20 | 0.20 | 0.20 |
| Red No. 201 | 0.80 | 0.80 | 0.80 | 0.80 | 0.80 | 0.80 |
| Red No. 202 | 0.80 | 0.80 | 0.80 | 0.80 | 0.80 | 0.80 |
| Polyglyceryl-2 triisostearate | 0.75 | 0.75 | 0.75 | 0.75 | 0.75 | 0.75 |
| Colcothar | 0.75 | 0.75 | 0.75 | 0.75 | 0.75 | 0.75 |
| Hydrophobic surface-treated titanium oxide | 2.20 | 2.20 | 2.20 | 2.20 | 2.20 | 2.20 |
| Copolymer 1 | 30.00 | | | | | |
| Copolymer 2 | | 30.00 | | | | |
| Copolymer 3 | | | 30.00 | | | |
| Copolymer 4 | | | | 20.00 | | |
| Copolymer 5 | | | | | 15.00 | |
| Copolymer 6 | | | | | | 10.00 |
| Glyceryl tri(caprylate/caprate) | Balance | Balance | Balance | Balance | Balance | Balance |
| Total | 100 | 100 | 100 | 100 | 100 | 100 |

**Table 31**

| Lipstick 4 | | | | | | |
|---|---|---|---|---|---|---|
| | Formulation Example 175 | Formulation Example 176 | Formulation Example 177 | Formulation Example 178 | Formulation Example 179 | Formulation Example 180 |
| Candelilla wax | 16.00 | 16.00 | 16.00 | 16.00 | 16.00 | 16.00 |
| Behenyl behenate | 4.00 | 4.00 | 4.00 | 4.00 | 4.00 | 4.00 |
| Diisostearyl malate | 15.00 | 15.00 | 15.00 | 15.00 | 15.00 | 15.00 |
| Triethylhexanoin | 8.00 | 8.00 | 8.00 | 8.00 | 8.00 | 8.00 |
| Propylparaben | 0.20 | 0.20 | 0.20 | 0.20 | 0.20 | 0.20 |
| Red No. 201 | 0.80 | 0.80 | 0.80 | 0.80 | 0.80 | 0.80 |
| Red No. 202 | 0.80 | 0.80 | 0.80 | 0.80 | 0.80 | 0.80 |
| Polyglyceryl-2 triisostearate | 0.75 | 0.75 | 0.75 | 0.75 | 0.75 | 0.75 |
| Colcothar | 0.75 | 0.75 | 0.75 | 0.75 | 0.75 | 0.75 |
| Hydrophobic surface-treated titanium oxide | 2.20 | 2.20 | 2.20 | 2.20 | 2.20 | 2.20 |
| Copolymer 1 | 30.00 | | | | | |
| Copolymer 2 | | 30.00 | | | | |
| Copolymer 3 | | | 30.00 | | | |
| Copolymer 4 | | | | 20.00 | | |
| Copolymer 5 | | | | | 15.00 | |
| Copolymer 6 | | | | | | 10.00 |
| Glyceryl tri(caprylate/caprate) | Balance | Balance | Balance | Balance | Balance | Balance |
| Total | 100 | 100 | 100 | 100 | 100 | 100 |

**Table 32**

| Lipstick 5 | | | | | | |
|---|---|---|---|---|---|---|
| | Formulation Example 181 | Formulation Example 182 | Formulation Example 183 | Formulation Example 184 | Formulation Example 185 | Formulation Example 186 |
| Dibutyl lauroyl glutamide | 3.00 | 3.00 | 3.00 | 3.00 | 3.00 | 3.00 |
| Dibutyl ethylhexanoyl glutamide | 2.00 | 2.00 | 2.00 | 2.00 | 2.00 | 2.00 |
| Glyceryl tri(caprylate/caprate) | 15.00 | 15.00 | 15.00 | 15.00 | 15.00 | 15.00 |
| Triethylhexanoin | 9.80 | 9.80 | 9.80 | 9.80 | 9.80 | 9.80 |
| Propylparaben | 0.20 | 0.20 | 0.20 | 0.20 | 0.20 | 0.20 |
| Red No. 218 | 0.50 | 0.50 | 0.50 | 0.50 | 0.50 | 0.50 |
| Copolymer 1 | 50.00 | | | | | |
| Copolymer 2 | | 50.00 | | | | |
| Copolymer 3 | | | 50.00 | | | |
| Copolymer 4 | | | | 40.00 | | |
| Copolymer 5 | | | | | 30.00 | |
| Copolymer 6 | | | | | | 20.00 |
| Diisostearyl malate | Balance | Balance | Balance | Balance | Balance | Balance |
| Total | 100 | 100 | 100 | 100 | 100 | 100 |

**Table 33**

| Mascara 2 | | | | | | |
|---|---|---|---|---|---|---|
| | Formulation Example 187 | Formulation Example 188 | Formulation Example 189 | Formulation Example 190 | Formulation Example 191 | Formulation Example 192 |
| Paraffin | 10.00 | 10.00 | 10.00 | 10.00 | 10.00 | 10.00 |
| Carnauba wax | 5.00 | 5.00 | 5.00 | 5.00 | 5.00 | 5.00 |
| Polyethylene | 4.00 | 4.00 | 4.00 | 4.00 | 4.00 | 4.00 |
| Microcrystalline wax | 1.00 | 1.00 | 1.00 | 1.00 | 1.00 | 1.00 |
| Phenoxyethanol | 0.50 | 0.50 | 0.50 | 0.50 | 0.50 | 0.50 |
| Propylparaben | 0.20 | 0.20 | 0.20 | 0.20 | 0.20 | 0.20 |
| Disteardimonium hectorite | 2.00 | 2.00 | 2.00 | 2.00 | 2.00 | 2.00 |
| Silicone surface-treated black iron oxide | 10.00 | 10.00 | 10.00 | 10.00 | 10.00 | 10.00 |
| Trimethylsiloxysilicic acid | 6.00 | 6.00 | 6.00 | 6.00 | 6.00 | 6.00 |
| Nylon-6 | 1.00 | 1.00 | 1.00 | 1.00 | 1.00 | 1.00 |
| Silica | 0.02 | 0.02 | 0.02 | 0.02 | 0.02 | 0.02 |
| Carbon black | 0.01 | 0.01 | 0.01 | 0.01 | 0.01 | 0.01 |
| Copolymer 1 | 5.00 | | | | | |
| Copolymer 2 | | 5.00 | | | | |
| Copolymer 3 | | | 5.00 | | | |
| Copolymer 4 | | | | 5.00 | | |
| Copolymer 5 | | | | | 5.00 | |
| Copolymer 6 | | | | | | 5.00 |
| Isododecane | Balance | Balance | Balance | Balance | Balance | Balance |
| Total | 100 | 100 | 100 | 100 | 100 | 100 |

The invention disclosed herein is as described below.
[A] A copolymer obtained by causing a diglycerin fatty acid ester represented by the following general formula (1) and isophorone diisocyanate to react with each other, the copolymer having a weight-average molecular weight of from 3,500 to 100,000: 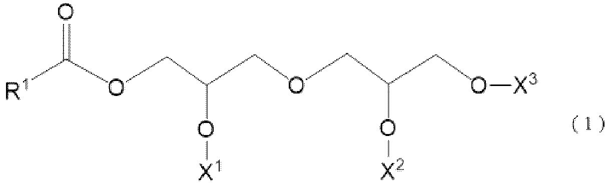 where R¹ represents an alkyl group having 8 to 24 carbon atoms or an alkenyl group having 8 to 24 carbon atoms, X¹ to X³ each independently represent a hydrogen atom or a group represented by -C(=O)R², and R² represents an alkyl group having 8 to 24 carbon atoms or an alkenyl group having 8 to 24 carbon atoms, provided that at least one of X¹ to X³ represents a hydrogen atom.
[B] The copolymer according to the item [A], wherein the diglycerin fatty acid ester comprises a diglycerin fatty acid ester represented by the general formula (1) in which X¹ and X² each represent a hydrogen atom, X³ represents a group represented by -C(=O)R², and R² represents an alkyl group having 8 to 24 carbon atoms or an alkenyl group having 8 to 24 carbon atoms.
[C] The copolymer according to the item [A] or [B], wherein the diglycerin fatty acid ester is a diglycerin fatty acid ester derived from a plant raw material.
[D] The copolymer according to any one of the items [A] to [C], wherein the weight-average molecular weight is from 5,000 to 40,000.
[E] A cosmetic composition, comprising the copolymer of any one of the items [A] to [D].
[F] The cosmetic composition according to the item [E], wherein a content of the copolymer is from 0.01 mass% to 90 mass%.

## Claims

1. A copolymer obtained by causing a diglycerin fatty acid ester represented by the following general formula (1) and isophorone diisocyanate to react with each other, the copolymer having a weight-average molecular weight of from 3,500 to 100,000: 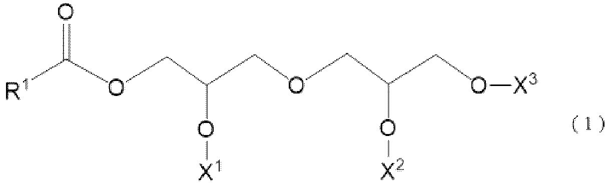 where R¹ represents an alkyl group having 8 to 24 carbon atoms or an alkenyl group having 8 to 24 carbon atoms, X¹ to X³ each independently represent a hydrogen atom or a group represented by -C(=O)R², and R² represents an alkyl group having 8 to 24 carbon atoms or an alkenyl group having 8 to 24 carbon atoms, provided that at least one of X¹ to X³ represents a hydrogen atom.

2. The copolymer according to claim 1, wherein the diglycerin fatty acid ester comprises a diglycerin fatty acid ester represented by the general formula (1) in which X¹ and X² each represent a hydrogen atom, X³ represents a group represented by -C(=O)R², and R² represents an alkyl group having 8 to 24 carbon atoms or an alkenyl group having 8 to 24 carbon atoms.

3. The copolymer according to claim 1, wherein the diglycerin fatty acid ester is a diglycerin fatty acid ester derived from a plant raw material.

4. The copolymer according to claim 1, wherein the weight-average molecular weight is from 5,000 to 40,000.

5. A cosmetic composition, comprising the copolymer of any one of claims 1 to 4.

6. The cosmetic composition according to claim 5, wherein a content of the copolymer is from 0.01 mass% to 90 mass%.
